# EUROPEAN PATENT SPECIFICATION

(11) **EP 2 087 117 B1**
(45) Date of publication and mention of the grant of the patent: **15.09.2010**
(21) Application number: 07822517.4
(22) Date of filing: 13.11.2007
(51) Int. Cl.: C12N 15/54, C12N 9/10, C12N 15/82, C12P 23/00, A23K 1/18

(54) **NEW KETOLASES FOR THE PRODUCTION OF KETOCAROTENOIDS IN TAGETES**
NEUE KETOLASEN ZUR HERSTELLUNG VON KETOCAROTENOIDEN IN TARGETS
NOUVELLES CÉTOLASES UTILISÉES POUR PRODUIRE DES CÉTOCAROTÉNOÏDES DANS DES TAGÈTES

(30) Priority: 15.11.2006 EP 06124146
(43) Date of publication of application: 12.08.2009
(73) Proprietor: BASF Plant Science GmbH, 67056 Ludwigshafen (DE)
(72) Inventor: SAUER, George Mather, 06484 Quedlinburg (DE); FLACHMANN, Ralf, 06484 Quedlinburg (DE); SCHOPFER, Christel Renate, 67061 Ludwigshafen (DE); LEPS, Michael, 38820 Halberstadt (DE); BRIDG-GIANNAKOPOULOS, Hannia, 06484 Quedlinburg (DE)
(74) Representative: Popp, Andreas
(86) International application number: PCT/EP2007/062241
(87) International publication number: WO 2008/058946

(56) References cited:
- WO-A-2005/019460
- US-A1- 2004 003 430
- HUANG JUN-CHAO ET AL: "Isolation and characterization of a carotenoid oxygenase gene from Chlorella zofingiensis (Chlorophyta)." APPLIED MICROBIOLOGY AND BIOTECHNOLOGY JUL 2006, vol. 71, no. 4, July 2006 (2006-07), pages 473-479, XP002476010 ISSN: 0175-7598
- MENG CHUN-XIAO ET AL: "Cloning and characterization of beta-carotene ketolase gene promoter in Haematococcus pluvialis." ACTA BIOCHIMICA ET BIOPHYSICA SINICA APR 2005, vol. 37, no. 4, April 2005 (2005-04), pages 270-275, XP002476011 ISSN: 1672-9145
- MANN V ET AL: "Metabolic engineering of astaxanthin production in tobacco flowers" NATURE BIOTECHNOLOGY, NATURE PUBLISHING GROUP, NEW YORK, NY, US, vol. 18, August 2000 (2000-08), pages 888-892, XP002969628 ISSN: 1087-0156

## Description

The present invention relates to new ketolases, their use for the expression of keto-carotenoids in plants preferably for the flower-specific expression, of genes in plants of the genus Tagetes, and to a process for the preparation of keto-carotenoids by culturing the genetically modified plants.

Various biosynthetic products, such as, for example, fine chemicals, such as, inter alia, amino acids, vitamins, carotenoids, but also proteins, are prepared in cells by means of natural metabolic processes und are used in many branches of industry, including the foodstuffs, feedstuffs, cosmetic, feed, food and pharmaceutical industries.

These substances, which together are described as fine chemicals/proteins, comprise, inter alia, organic acids, both proteinogenic and nonproteinogenic amino acids, nucleotides and nucleosides, lipids and fatty acids, diols, carbohydrates, aromatic compounds, vitamins, carotenoids and cofactors, and also proteins and enzymes. Their production on the large-scale in some cases takes place by means of biotechnological processes using microorganisms which were developed in order to produce and secrete large amounts of the in each case desired substance.

Carotenoids are synthesized de novo in bacteria, algae, fungi and plants. In recent years, it has increasingly been attempted also to utilize plants as production organisms for fine chemicals, in particular for vitamins and carotenoids.

A natural mixture of the carotenoids lutein, zeaxanthin and violaxanthin is extracted, for example, from the flowers of marigold plants (Tagetes plants) as "oleoresin". This oleoresin is used both as an ingredient of food supplements and in the feed sector.

Lycopene from tomatoes is likewise used as a food supplement, while phytoene is mainly used in the cosmetic sector.

Ketocarotenoids, that is carotenoids which comprise at least one keto group, such as, for example, astaxanthin, canthaxanthin, echinenone, 3-hydroxyechinenone, 3'-hydroxyechinenone, adonirubin and adonixanthin are natural antioxidants and pigments which are produced by some algae, plants and microorganisms as secondary metabolites.

On account of their color-imparting properties, the ketocarotenoids and in particular astaxanthin are used as pigmenting aids in animal nutrition, in particular in trout, salmon and shrimp farming.

An economical biotechnological process for the production of natural, biosynthetic products and in particular carotenoids is therefore of great importance.

WO 0032788 describes some carotenoid biosynthesis genes from plants of the genus Tagetes and discloses how genetically modified plants of the genus Tagetes could be produced in order to obtain various carotenoid profiles in the petals and thus to produce certain carotenoids selectively. To this end, it is necessary to overexpress some biosynthesis genes and to suppress others.

For the overexpression of the newly found carotenoid biosynthesis genes in plants of the genus Tagetes, WO 0032788 postulates the petal-specific promoter of the ketolase from Adonis vemalis.

The ketolases used so far in the prior art do not meet all requirements which are necessary in order to effect a high expression of keto-carotenoids in Tagetes

The invention therefore relates to the use of new ketolases having a polypeptide sequence SEQ ID NO:2 or SEQ ID NO:6, a sequence derived from SEQ ID NO:2 or SEQ ID NO:6 by substitution, insertion or deletion of aminoacids, which has an identity of at least 60% with the respective sequence SEQ. ID NO:2 or SEQ ID NO:6 , for the expression of heterologous genes in lutein-depleted

A "ketolase" is understood as meaning a protein which has the enzymatic activity to introduce a keto group on the optionally substituted beta-ionone ring of carotenoids. This enzymatic activity is meant when the term "functionally equivalent fragment" is used in connection with ketolase polypeptides.

In particular, a ketolase is understood as meaning a protein which has the enzymatic activity to convert beta-carotene to canthaxanthin.

Examples for amino acid substitutions starting from the SEQ ID NO: 1A or SEQ ID NO:4A are listed in the following table:

| Original residue | e.g. substituted by |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

A "promoter" is understood according to the invention as meaning a nucleic acid having expression activity, that is a nucleic acid which, in functional linkage with a nucleic acid to be expressed, also described as a gene below, regulates the expression, that is the transcription and the translation of this nucleic acid or this gene.

"Transcription" is understood according to the invention as meaning the process by which, starting from a DNA matrix, a complementary RNA molecule is prepared. Proteins such as RNA polymerase, "sigma factors" and transcriptional regulator proteins are involved in this process. The RNA synthesized is then used as a matrix in the translation process, which then leads to the biosynthetically active protein.

A "functional linkage" is understood in this connection as meaning, for example, the sequential arrangement of one of the promoters according to the invention and a nucleic acid sequence to be expressed and, if appropriate, further regulative elements such as, for example, a terminator in such a way that each of the regulative elements can fulfill its function in the expression of the nucleic acid sequence. To this end, a direct linkage in the chemical sense is not imperative. Genetic control sequences, such as, for example, enhancer sequences, can also exert their function on the target sequence from positions which are further removed or even from other DNA molecules. Arrangements are preferred in which the nucleic acid sequence to be expressed or the gene to be expressed is positioned behind (i.e. at the 3'-end) of the promoter sequence according to the invention, such that both sequences are bonded covalently to one another. Preferably, the distance between the promoter sequence and the nucleic acid sequence to be expressed is in this case lower than 200 base pairs, particularly preferably less than 100 base pairs, very particularly preferably less than 50 base pairs.

"Expression activity" is understood according to the invention as meaning the amount of protein formed in a certain time by the promoter, that is the expression rate.

"Specific expression activity" is understood according to the invention as meaning the amount of protein per promoter formed in a certain time by the promoter.

In the case of a "caused expression activity" or "caused expression rate" in relation to a gene in comparison with the wild-type, in comparison with the wild-type the formation of a protein is thus caused which thus was not present in the wild-type.

In the case of an "increased expression activity" or "increased expression rate" in relation to a gene in comparison with the wild-type, in comparison with the wild-type the amount of protein formed in a certain time is thus increased.

The formation rate at which a biosynthetically active protein is prepared is a product of the rate of transcription and translation. Both rates can be influenced according to the invention and thus influence the rate of formation of products in a microorganism and plant.

"Heterologous gene expression " means according to the invention that a promoter and the functionally linked gene do not exist in such a construction in wild-types plants. Heterologous gene expression encompasses the cases the promoter or the gene or both components do not exist in the wild-type of the respective plant or where the promoter and the gene do exist in the wildtype but at different genetic locations which do not allow a functional linkage in the wildtype.

The term "wild-type" or "wild-type plant" is understood according to the invention as meaning the corresponding starting plant of the genus Tagetes.

Depending on the connection, the term "plant" can be understood as meaning the starting plant (wild-type) or a genetically modified plant according to the invention of the genus Tagetes or both.

Preferably, "wild-type" is understood as meaning the plant Tagetes erecta, in particular the plant Tagetes erecta Hybrid 50011 (WO 02012438) as well as Tagetes erecta 13819 and the derivatives produced by mutagenesis or selection as a reference organism for the increasing or causing of the expression activity or expression rate and for the increasing of the content of biosynthetic products.

Another embodiment of the invention are polynucleotide sequences encoding ketolases having a polypeptide sequence SEQ ID NO:2 or SEQ ID NO:6, a sequence derived from SEQ ID NO:2 or SEQ ID NO:6 by substitution, insertion or deletion of aminoacids, which has an identity of at least 60% with the respective sequence SEQ. ID NO:2 or SEQ ID NO:6.

These polynucleotide sequences can be generated by a retranslation of the polypeptide sequence according to the genetic code. A preferred polynucleotide sequence is adapted in its codon usage to the host organism in which the polynucleotide sequence shall be expressed. All abovementioned polynucleotide sequences can be prepared in a manner known per se by chemical synthesis from the nucleotide structural units such as, for example, by fragment condensation of individual overlapping, complementary nucleic acid structural units of the double helix. The chemical synthesis of oligonucleotides can be carried out, for example, in a known manner, according to the phosphoamidite method (Voet, Voet, 2nd edition, Wiley Press New York, pp. 896-897). The addition of synthetic oligonucleotides and filling of gaps with the aid of the Klenow fragment of the DNA polymerase and ligation reactions and general cloning processes are described in Sambrook et al. (1989), Molecular cloning: A laboratory manual, Cold Spring Harbor Laboratory Press.

A preferred embodiment of the invention are polynucleotide sequences encoding ketolases wherein the polynucleotide sequences comprise sequences pursuant SEQ ID NO. 2 or SEQ ID NO:5.

A preferred embodiment of the invention are polynucleotide sequences encoding ketolases wherein this polynucleotide sequences are under the transcriptional control of a plastid-lipid associated protein promoter (PAP-promoter)

An "PAP- promoter" is understood as meaning promoters which naturally do exist preferably in plans such as cucumber, tomato and rape and regulate the gene expression of plastid-lipid associated proteins.

Preferred PAP promoters comprise
A1) the nucleic acid sequence SEQ. ID. NO:10 or 11 or
A2) a sequence derived from these sequences by substitution, insertion or deletion of nucleotides, which has an identity of at least 60% at nucleic acid level with the respective sequence SEQ. ID. NO:10 or 11 or
A3) a nucleic acid sequence which is hybridized with the nucleic acid sequence SEQ. ID. NO:10 or 11 under stringent conditions or
A4) functionally equivalent fragments of the sequences under A1), A2) or A3)

The nucleic acid sequence SEQ. ID. NO. 10 represents a promoter sequence of hypothetical plastid-lipid associated protein from Brassica napus.

The nucleic acid sequence SEQ. ID. NO. 11 represents a promoter sequence of hypothetical plastid-lipid associated protein from Brassica napus.

The invention furthermore relates to PAP promoters, comprising a sequence derived from these sequences (SEQ. ID. NO:10 or 11) by substitution, insertion or deletion of nucleotides, which has an identity of at least 60% at nucleic acid level with the respective sequence SEQ. ID. NO:10 or 11.

Further natural examples according to the invention of PAP promoters according to the invention can easily be found, for example, from various organisms whose genomic sequence is known, by identity comparisons of the nucleic acid sequences from databases containing the sequences SEQ ID NO:10 or 11 described above.

Synthetic PAP promoter sequences according to the invention can easily be found starting from the sequences SEQ ID NO: 10 or 11 by synthetic variation and mutation, for example by substitution, insertion or deletion of nucleotides.

The following definitions and conditions of the identity comparisons and hybridization conditions apply for all nucleic acids, that is all promoters and genes of the description.

The term "substitution" is to be understood as meaning the exchange of one or more nucleotides for one or more nucleotides. "Deletion" is the replacement of a nucleotide by a direct bond. Insertions are insertions of nucleotides into the nucleic acid sequence, a direct bond formally being replaced by one or more nucleotides.

Identity between two nucleic acids is understood as meaning the identity of the nucleotides over the entire nucleic acid length in each case, in particular the identity which is calculated by comparison with the aid of the Vector NTI Suite 7.1 software of Informax (USA) using the Clustal method (Higgins DG, Sharp PM. Fast and sensitive multiple sequence alignments on a microcomputer. Comput Appl. Biosci. 1989 Apr;5(2):151-1) with setting of the following parameters:
Multiple alignment parameter:
   Gap opening penalty 10
   Gap extension penalty 10
   Gap separation penalty range 8
   Gap separation penalty off
   % identity for alignment delay 40
   Residue specific gaps off
   Hydrophilic residue gap off
   Transition weighing 0
Pairwise alignment parameter:
   FAST algorithm on
   K-tuple size 1
   Gap penalty 3
   Window size 5
   Number of best diagonals 5

A nucleic acid sequence which has an identity of at least 60% with the sequence SEQ ID NO: N is accordingly understood as meaning a nucleic acid sequence which, in a comparison of its sequence with the sequence SEQ ID NO: N, in particular according to the above program logarithm, has an identity of at least 60% with the above parameter set.

Particularly preferred PAP promoters have, with the respective nucleic acid sequence SEQ. ID. NO. 10 or 11, an identity of at least 70%, more preferably at least 80%, at least 90%, at least 92%, at least 95%, at least 96%, at least 97%, at least 98%, particularly preferably at least 99%.

Further natural examples of PAP promoters can furthermore easily be found starting from the nucleic acid sequences described above, in particular starting from the sequences SEQ ID NO: 10 or 11 , of various organisms whose genomic sequence is not known, by hybridization techniques in a manner known per se.

A further subject of the invention therefore relates to PAP promoters comprising a nucleic acid sequence which hybridizes with the nucleic acid sequence SEQ. ID. No.10 or 11 under stringent conditions. This nucleic acid sequence comprises at least 10, more preferably more than 12, 15, 30, 50 or particularly preferably more than 150 nucleotides.

"Hybridization" is understood as meaning the ability of a poly- or oligonucleotide to bind under stringent conditions to a nearly complementary sequence, while under these conditions nonspecific binding between noncomplementary partners does not happen. To this end, the sequences should preferably be complementary to 90-100%. The characteristic of complementary sequences to be able to bind specifically to one another is made use of, for example, in the Northern or Southern blot technique or in primer binding in PCR or RT-PCR.

Hybridization takes place according to the invention under stringent conditions. Such hybridization conditions are described, for example, in Sambrook, J., Fritsch, E.F., Maniatis, T., in: Molecular Cloning (A Laboratory Manual), 2nd edition, Cold Spring Harbor Laboratory Press, 1989, pages 9.31-9.57 or in Current Protocols in Molecular Biology, John Wiley & Sons, N.Y. (1989), 6.3.1-6.3.6:

Stringent hybridization conditions are in particular understood as meaning: Overnight incubation at 42°C in a solution consisting of 50% formamide, 5 x SSC (750 mM NaCl, 75 mM trisodium citrate), 50 mM sodium phosphate (ph 7.6), 5x Denhardt solution, 10% dextran sulfate and 20 g/ml of denatured, sheared salmon sperm DNA, followed by washing of the filter with 0.1 x SSC at 65°C.

For promoters, a "functionally equivalent fragment" is understood as meaning fragments essentially having the same promoter activity as the starting sequence.

"Essentially identical" is understood as meaning a specific expression activity which has at least 50%, preferably 60%, more preferably 70%, more preferably 80%, more preferably 90%, particularly preferably 95%, of the specific expression activity of the starting sequence.

"Fragments" are understood as meaning subsequences of the PAP promoters described by embodiment A1), A2) or A3). Preferably, these fragments have more than 10, but more preferably more than 12, 15, 30, 50 or particularly preferably more than 150, connected nucleotides of the nucleic acid sequence SEQ. ID. NO. 9, or 12.

The use of the nucleic acid sequence SEQ. ID. NO. 10 or 11 as an PAP promoter, i.e. for the expression of genes in plants of the genus Tagetes, which are depleted in their lutein productivity, is particularly preferred.

All abovementioned PAP promoters can furthermore be prepared in a manner known per se by chemical synthesis from the nucleotide structural units such as, for example, by fragment condensation of individual overlapping, complementary nucleic acid structural units of the double helix. The chemical synthesis of oligonucleotides can be carried out, for example, in a known manner, according to the phosphoamidite method (Voet, Voet, 2nd edition, Wiley Press New York, pp. 896-897). The addition of synthetic oligonucleotides and filling of gaps with the aid of the Klenow fragment of the DNA polymerase and ligation reactions and general cloning processes are described in Sambrook et al. (1989), Molecular cloning: A laboratory manual, Cold Spring Harbor Laboratory Press.

Using the promoters according to the invention, in principle any gene, that is any nucleic acid, encoding a protein, can be expressed in plants of the genus Tagetes, in particular expressed flower-specifically, particularly preferably expressed petal-specifically.

These genes to be expressed in plants of the genus Tagetes are also called "effect genes" below.

Preferred effect genes are, for example, genes from the biosynthesis pathway of odoriferous substances and flower colors whose expression or increased expression in plants of the genus Tagetes leads to a change in the odor and/or the flower color of flowers of the plants of the genus Tagetes.

The biosynthesis of volatile odor components, especially in flowers, was in recent years studied on various model organisms such as Clarkia breweri and Antirhinum majus L. Volatile odor components are formed, for example, within the monoterpene and phenylpropane metabolism. In the first case, the component is linalool; methyleneeugenol, benzyl acetate, methyl benzoate and methyl salicate are derived from the phenylpropanes.

For the biosynthesis of linalool, (Iso)methyleigenol, benzyl acetate and methyl salicinate, preferred genes are selected from the group consisting of nucleic acids encoding a linalool synthase (LIS), nucleic acids encoding an S-adenosyl-L-Met:(iso)-eugenol O-methyltransferase (IEMT), nucleic acids encoding an acetyl-CoA-benzyl alcohol acetyltransferase and nucleic acids encoding an S-adenosyl-L-Met: salicylic acid methyltransferase (SAMT). Nucleic acid sequences and protein sequences for the enzymatic activities mentioned are described in Dudareva et al. Plant Cell 8 (1996), 1137-1148; Wang et al. Plant Physiol. 114 (1997), 213-221 and Dudareva et al. Plant J. 14 (1998) 297-304.

Particularly preferred effect genes are genes from biosynthesis pathways of biosynthetic products which can be prepared in plants of the genus Tagetes naturally, i.e. in the wild-type or by genetic modification of the wild-type, in particular can be prepared in flowers, particularly preferably can be prepared in petals.

Lutein-depleted Tagetes is a Tagetes plant, where the production of lutein has been decreased. The production of such lutein-depleted Tagetes has been disclosed in US 6,784,351 which is herewith incorporated by reference entirely.

Preferred biosynthetic products are fine chemicals.

The term "fine chemical" is known in the specialist field and comprises compounds which are produced by an organism and are applied in various branches of industry, such as, for example, but not restricted to, the pharmaceutical industry, the agricultural industry, cosmetics, food and feed industries. These compounds comprise organic acids, such as, for example, tartaric acid, itaconic acid and diaminopimelic acid, both proteinogenic and nonproteinogenic amino acids, purine and pyrimidine bases, nucleosides and nucleotides (as described, for example, in Kuninaka, A. (1996) Nucleotides and related compounds, pp. 561-612, in Biotechnology vol. 6, Rehm et al., publ. VCH: Weinheim and the citation contained therein), lipids, saturated and unsaturated fatty acids (for example arachidonic acid), diols (for example propanediol and butanediol), carbohydrates (for example hyaluronic acid and trehalose), aromatic compounds (for example aromatic amines, vanillin and indigo), vitamins, carotenoids and cofactors (as described in Ullmann's Encyclopedia of Industrial Chemistry, vol. A27, "Vitamins", pp. 443-613 (1996) VCH: Weinheim and the citation contained therein; and Ong, A.S., Niki, E. and Packer, L. (1995) "Nutrition, Lipids, Health and Disease" Proceedings of the UNESCO/Confederation of Scientific and Technological Associations in Malaysia and the Society for Free Radical Research - Asia, held on Sept. 1 st-3rd 1994 in Penang, Malysia, AOCS Press (1995)), enzymes and all other chemicals described by Gutcho (1983) in Chemicals by Fermentation, Noyes Data Corporation, ISBN: 0818805086 and the references stated therein. The metabolism and the uses of certain fine chemicals are further illustrated below.

### I. Amino acid metabolism and uses

The amino acids comprise the fundamental structural units of all proteins and are thus essential for the normal cell functions. The term "amino acid" is known in the specialist field. The proteinogenic amino acids, of which there are 20 types, serve as structural units for proteins, in which they are linked to one another by means of peptide bonds, whereas the nonproteinogenic amino acids (of which hundreds are known) usually do not occur in proteins (see Ullmann's Encyclopedia of Industrial Chemistry, vol. A2, pp. 57-97 VCH: Weinheim (1985)). The amino acids can be present in the D- or L-configuration, although L-amino acids are usually the only type which are found in naturally occurring proteins. Biosynthesis and degradation pathways of each of the 20 proteinogenic amino acids are well characterized both in prokaryotic and eukaryotic cells (see, for example, Stryer, L. Biochemistry, 3rd edition, pp. 578-590 (1988)). The "essential" amino acids (histidine, isoleucine, leucine, lysine, methionine, phenylalanine, threonine, tryptophan and valine), so-called as on account of the complexity of their biosynthesis they have to be assimilated with the food, are converted by means of simple biosynthesis pathways into the other 11 "nonessential" amino acids (alanine, arginine, asparagine, aspartate, cysteine, glutamate, glutamine, glycine, proline, serine and tyrosine). Higher animals possess the ability to synthesize some of these amino acids, but the essential amino acids have to be assimilated with the food in order that normal protein synthesis takes place.

Apart from their function in protein biosynthesis, these amino acids are interesting chemicals per se, and it has been discovered that many are used in various applications in the foodstuffs, feedstuffs, chemical, cosmetics, agriculture and pharmaceutical industries. Lysine is an important amino acid not only for the nutrition of humans, but also for monogastric animals, such as poultry and pigs. Glutamate is most often used as a flavor additive (monosodium glutamate, MSG) and to a great extent in the foodstuffs industry, as are also aspartate, phenylalanine, glycine and cysteine. Glycine, L-methionine and tryptophan are all used in the pharmaceutical industry. Glutamine, valine, leucine, isoleucine, histidine, arginine, proline, serine and alanine are used in the pharmaceutical industry and the cosmetics industry. Threonine, tryptophan and D-/L-methionine are widespread feedstuff additives (Leuchtenberger, W. (1996) Amino acids - technical production and use, pp. 466-502 in Rehm et al., (ed.) Biotechnology vol. 6, chapter 14a, VCH: Weinheim). It has been discovered that these amino acids are moreover suitable as precursors for the synthesis of synthetic amino acids and proteins, such as N-acetylcysteine, S-carboxymethyl-L-cysteine, (S)-5-hydroxytryptophan and other substances, described in Ullmann's Encyclopedia of Industrial Chemistry, vol. A2, pp. 57-97, VCH, Weinheim, 1985.

The biosynthesis of these natural amino acids in organisms which can produce them, for example bacteria, has been well characterized (for a general survey of bacterial amino acid biosynthesis and its regulation, see Umbarger, H.E. (1978) Ann. Rev. Biochem. 47: 533 - 606). Glutamate is synthesized by reductive animation of - ketoglutarate, an intermediate in the citric acid cycle. Glutamine, proline and arginine are in each case produced in succession from glutamate. The biosynthesis of serine takes place in a three-step process and begins with 3-phosphoglycerate (an intermediate in glycolysis), and affords this amino acid after oxidation, transamination and hydrolysis steps. Cysteine and glycine are in each case produced from serine, namely the former by condensation of homocysteine with serine, and the latter by transfer of the side chain alpha-carbon atom to tetrahydrofolate, in a reaction catalyzed by serine transhydroxymethylase. Phenylalanine and tyrosine are synthesized from the precursors of the glycolysis and pentose phosphate pathway, erythrose 4-phosphate and phosphoenolpyruvate in a 9-step biosynthesis pathway, which only differs in the last two steps after the synthesis of prephenate. Tryptophan is likewise produced from these two starting molecules, but its synthesis takes place in an 11-step pathway. Tyrosine can also be prepared from phenylalanine in a reaction catalyzed by phenylalanine hydroxylase. Alanine, valine and leucine are in each case biosynthesis products of pyruvate, the end product of the glycolysis. Aspartate is formed from oxalacetate, an intermediate of the citrate cycle. Asparagine, methionine, threonine and lysine are in each case formed by conversion of aspartate. Isoleucine is formed from threonine. In a complex 9-step pathway, the formation of histidine takes place from 5-phosphoribosyl-1-pyrophosphate, an activated sugar.

Amino acids whose amount exceeds the protein biosynthesis need of the cell cannot be stored, and instead of this are broken down, so that intermediates for the main metabolic pathways of the cell are made available (for a general survey see Stryer, L., Biochemistry, 3rd ed. chap. 21 "Amino Acid Degradation and the Urea Cycle"; pp 495-516 (1988)). Although the cell is able to convert undesired amino acids to useful metabolic intermediates, the amino acid production with respect to the energy, the precursor molecules and the enzymes necessary for their synthesis is complicated. It is therefore not surprising that amino acid biosynthesis is regulated by feedback inhibition, the presence of a certain amino acid slowing or completely ending its own production (for a general survey of the feedback mechanism in amino acid biosynthesis pathways, see Stryer, L., Biochemistry, 3rd ed., chap. 24, "Biosynthesis of Amino Acids and Heme", pp. 575-600 (1988)). The output of a certain amino acid is therefore restricted by the amount of this amino acid in the cell.

### II. Vitamins, carotenoids, cofactors and nutraceuticals-metabolism and uses

Vitamins, carotenoids, cofactors and nutraceuticals comprise a further group of molecules. Higher animals have lost the ability to synthesize these and must thus assimilate them, although they are easily synthesized by other organisms, such as bacteria. These molecules are either biologically active molecules per se or precursors of biologically active substances, which serve as electron carriers or intermediates in a number of metabolic pathways. In addition to their nutritional value, these compounds also have a significant industrial value as colorants, antioxidants and catalysts or other processing aids. (For a general survey on the structure, activity and the industrial applications of these compounds see, for example, Ullmann's Encyclopedia of Industrial Chemistry, "Vitamins", vol. A27, pp. 443-613, VCH: Weinheim, 1996). The term "vitamin" is known in the specialist field and comprises nutrients which are needed by an organism for normal function, but cannot be synthesized by this organism itself. The group consisting of the vitamins can comprise cofactors and nutraceutical compounds. The term "cofactor" comprises nonprotein-like compounds, which are necessary for the occurrence of normal enzyme activity. These compounds can be organic or inorganic; the cofactor molecules according to the invention are preferably organic. The term "nutraceutical" comprises food additives which are health-promoting in plants and animals, in particular humans. Examples of such molecules are vitamins, antioxidants and also certain lipids (e.g. polyunsaturated fatty acids).

Preferred fine chemicals or biosynthetic products which can be prepared in plants of the genus Tagetes, in particular in petals of the flowers of the plants of the genus Tagetes, are carotenoids, such as, for example, phytoene, lycopene, lutein, zeaxanthin, astaxanthin, canthaxanthin, echinenone, 3-hydroxyechinenone, 3'-hydroxyechinenone, adonirubin, violaxanthin and adonixanthin.

Particularly preferred carotenoids are ketocarotenoids, such as, for example, astaxanthin, canthaxanthin, echinenone, 3-hydroxyechinenone, 3'-hydroxyechinenone, adonirubin and adonixanthin.

The biosynthesis of these molecules in organisms which are capable of their production, such as bacteria, has been comprehensively characterized (Ullmann's Encyclopedia of Industrial Chemistry, "Vitamins", vol. A27, pp. 443-613, VCH: Weinheim, 1996, Michal, G. (1999) Biochemical Pathways: An Atlas of Biochemistry and Molecular Biology, John Wiley & Sons; Ong, A.S., Niki, E. and Packer, L. (1995) "Nutrition, Lipids, Health and Disease" Proceedings of the UNESCO/Confederation of Scientific and Technological Associations in Malaysia and the Society for free Radical Research - Asia, held on Sept. 1 st-3rd 1994 in Penang, Malaysia, AOCS Press, Champaign, IL X, 374 S).

Thiamine (vitamin B₁) is formed by chemical coupling of pyrimidine and thiazole units. Riboflavin (vitamin B₂) is synthesized from guanosine 5'-triphosphate (GTP) and ribose 5'-phosphate. Riboflavin is in turn employed for the synthesis of flavine mononucleotide (FMN) and flavine adenine dinucleotide (FAD) . The family of compounds which together are described as "vitamin B6" (for example pyridoxine, pyridoxamine, pyridoxal 5'-phosphate and the commercially used pyridoxine hydrochloride), are all derivatives of the common structural unit 5-hydroxy-6-methylpyridine. Panthothenate (pantothenic acid, R-(+)-N-(2,4-dihydroxy-3,3-dimethyl-1-oxobutyl)-f3-alanine) can be prepared either by chemical synthesis or by fermentation. The last steps in pantothenate biosynthesis consist of the ATP-driven condensation of β-alanine and pantoic acid. The enzymes responsible for the biosynthesis steps for the conversion to pantoic acid, to β-alanine and for the condensation to pantothenic acid are known. The metabolically active form of pantothenate is coenzyme A, whose biosynthesis proceeds via 5 enzymatic steps. Pantothenate, pyridoxal 5'-phosphate, cysteine and ATP are the precursors of coenzyme A. These enzymes catalyze not only the formation of pantothenate, but also the production of (R)-pantoic acid, (R)-pantolactone, (R)-panthenol (provitamin B₅), pantethein (and its derivatives) and coenzyme A.

The biosynthesis of biotin from the precursor molecule pimeloyl-CoA in microorganisms has been investigated in detail, and several of the genes involved have been identified. It has emerged that many of the corresponding proteins are involved in the Fe cluster synthesis and belong to the class of the nifS proteins. Lipoic acid is derived from octanoic acid and serves as a coenzyme in energy metabolism, where it is a constituent of the pyruvate dehydrogenase complex and of the alpha-ketoglutarate dehydrogenase complex. The folates are a group of substances which are all derived from folic acid, which in turn is derived from L-glutamic acid, p-amino-benzoic acid and 6-methylpterin. The biosynthesis of folic acid and its derivatives, starting from the metabolic intermediates guanosine 5'-triphosphate (GTP), L-glutamic acid and p-amino-benzoic acid, has been investigated in detail in certain microorganisms.

Corrinoids (such as the cobalamines and in particular vitamin B₁₂) and the porphyrins belong to a group of chemicals which are distinguished by a tetrapyrrole ring system. The biosynthesis of vitamin B₁₂ is sufficiently complex that it has still not been completely characterized, but in the meantime a majority of the enzymes and substrates involved is known. Nicotinic acid (nicotinate) and nicotinamide are pyridine derivatives which are also described as "niacin". Niacin is the precursor of the important coenzyme NAD (nicotinamide adenine dinucleotide) and NADP (nicotinamide adenine dinucleotide phosphate) and their reduced forms.

The production of these compounds on the large-scale is based in the main on cell-free chemical syntheses, although some of these chemicals have also been produced by large-scale culture of microorganisms, such as riboflavin, vitamin B₆, pantothenate and biotin. Only vitamin B₁₂ is produced solely by fermentation on account of the complexity of its synthesis. In-vitro processes require a considerable expenditure of materials and time and frequently of high costs.

### III. Purine, pyrimidine, nucleoside and nucleotide metabolism and uses

Genes for purine and pyrimidine metabolism and their corresponding proteins are important targets for the therapy of tumors and virus infections. The term "purine" or "pyrimidine" comprises nitrogen-containing bases, which are a constituent of the nucleic acids, coenzymes and nucleotides. The term "nucleotide" comprises the basic structural units of the nucleic acid molecules, which comprise a nitrogen-containing base, a pentose sugar (in RNA the sugar is ribose, in DNA the sugar is D-deoxyribose) and phosphoric acid. The term "nucleoside" comprises molecules which serve as precursors of nucleotides, but which in contrast to the nucleotides contain no phosphoric acid unit. By inhibition of the biosynthesis of these molecules or their mobilization for the formation of nucleic acid molecules it is possible to inhibit the RNA and DNA synthesis; if this activity is inhibited in cancerogenic cells in a targeted manner, the division and replication ability of tumor cells can be inhibited.

There are moreover nucleotides which form no nucleic acid molecules, but serve as energy stores (i.e. AMP) or as coenzymes (i.e. FAD and NAD).

Several publications have described the use of these chemicals for these medicinal indications, in which the purine and/or pyrimidine metabolism is influenced (for example Christopherson, R.I. and Lyons, S.D. (1990) "Potent inhibitors of de novo pyrimidine and purine biosynthesis as chemotherapeutic agents", Med. Res. Reviews 10: 505-548). Investigations on enzymes which are involved in purine and pyrimidine metabolism have concentrated on the development of new medicaments, which can be used, for example, as immunosuppressive agents or antiproliferants (Smith, J.L. "Enzymes in Nucleotide Synthesis" Curr. Opin. Struct. Biol. 5 (1995) 752-757; Biochem. Soc. Transact. 23 (1995) 877-902). The purine and pyrimidine bases, nucleosides and nucleotides, however, also have other use possibilities: as intermediates in the biosynthesis of various fine chemicals (e.g. thiamine, S-adenosylmethionine, folates or riboflavin), as energy sources for the cell (for example ATP or GTP) and, for chemicals themselves, are usually used as flavor enhancers (for example IMP or GMP) or for many medicinal applications (see, for example, Kuninaka, A., (1996) "Nucleotides and Related Compounds in Biotechnology vol. 6, Rehm et al., ed. VCH: Weinheim, pp. 561-612). Enzymes which are involved in purine, pyrimidine, nucleoside or nucleotide metabolism are also used more and more highly as targets against which chemicals for plant protection, including fungicides, herbicides and insecticides, are developed.

The metabolism of these compounds in bacteria has been characterized (for general surveys see, for example, Zalkin, H. and Dixon, J.E. (1992) "De novo purine nucleotide biosynthesis" in Progress in Nucleic Acids Research and Molecular biology, vol. 42, Academic Press, pp. 259-287; and Michal, G. (1999) "Nucleotides and Nucleosides"; chap. 8 in: Biochemical Pathways: An Atlas of Biochemistry and Molecular Biology, Wiley, New York). Purine metabolism, the subject of intensive research, is essential for the normal functioning of the cell. Defective purine metabolism in higher animals can cause serious disorders, for example gout. The purine nucleotides are synthesized from ribose 5-phosphate via a series of steps by means of the intermediate compound inosine 5'-phosphate (IMP), which leads to the production of guanosine 5'-monophosphate (GMP) or adenosine 5'-monophosphate (AMP), from which the triphosphate forms used as nucleotides can readily be prepared. These compounds are also used as energy stores, so that their breakdown supplies energy for many different biochemical processes in the cell. Pyrimidine biosynthesis takes place via the formation of uridine 5'-monophosphate (UMP) from ribose 5-phosphate. UMP in turn is converted to cytidine 5'-triphosphate (CTP). The deoxy forms of all nucleotides are prepared in a single-step reduction reaction of the diphosphate ribose form of the nucleotide to the diphosphate deoxyribose form of the nucleotide. After phosphorylation, these molecules can participate in DNA synthesis.

### IV. Trehalose metabolism and uses

Trehalose consists of two glucose molecules, which are linked to one another. It is usually used in the foodstuffs industry as a sweetener, as an additive for dried or frozen foods and in drinks. It is, however, also used in the pharmaceutical industry, the cosmetics and biotechnology industry (see, for example, Nishimoto et al., (1998) US Patent No. 5 759 610; Singer, M.A. and Lindquist, S. Trends Biotech. 16 (1998) 460-467; Paiva, C.L.A. and Panek, A.D. Biotech Ann. Rev. 2 (1996) 293-314; and Shiosaka, M. J. Japan 172 (1997) 97-102). Trehalose is produced by enzymes of many microorganisms and released naturally into the surrounding medium, from which it can be recovered by processes known in the specialist field.

Particularly preferred biosynthetic products are selected from the group consisting of organic acids, proteins, nucleotides and nucleosides, both proteinogenic and nonproteinogenic amino acids, lipids and fatty acids, diols, carbohydrates, aromatic compounds, vitamins and cofactors, enzymes and proteins.

Preferred organic acids are tartaric acid, itaconic acid and diaminopimelic acid.

Preferred nucleosides and nucleotides are described, for example, in Kuninaka, A. (1996) Nucleotides and related compounds, pp. 561-612, in Biotechnology vol. 6, Rehm et al., ed. VCH: Weinheim and the citations contained therein.

Preferred biosynthetic products are furthermore lipids, saturated and unsaturated fatty acids, such as, for example, arachidonic acid, diols such as, for example, propanediol and butanediol, carbohydrates, such as, for example, hyaluronic acid and trehalose, aromatic compounds, such as, for example, aromatic amines, vanillin and indigo, vitamins and cofactors, such as are described, for example, in Ullmann's Encyclopedia of Industrial Chemistry, vol. A27, "Vitamins", pp. 443-613 (1996) VCH: Weinheim and the citations comprised therein; and Ong, A.S., Niki, E. and Packer, L. (1995) "Nutrition, Lipids, Health and Disease" Proceedings of the UNESCO/Confederation of Scientific and Technological Associations in Malaysia and the Society for Free Radical Research - Asia, held on Sept. 1 st-3rd 1994 in Penang, Malysia, AOCS Press (1995)), enzyme polyketides (Cane et al. (1998) Science 282: 63-68), and all other chemicals described by Gutcho (1983) in Chemicals by Fermentation, Noyes Data Corporation, ISBN: 0818805086 and the references stated therein.

Particularly preferred genes which are expressed with the promoters according to the invention in plants of the genus Tagetes are accordingly genes selected are from the group consisting of nucleic acids encoding a protein from the biosynthesis pathway of proteinogenic and nonproteinogenic amino acids, nucleic acids encoding a protein from the biosynthesis pathway of nucleotides and nucleosides, nucleic acids encoding a protein from the biosynthesis pathway of organic acids, nucleic acids encoding a protein from the biosynthesis pathway of lipids and fatty acids, nucleic acids encoding a protein from the biosynthesis pathway of diols, nucleic acids encoding a protein from the biosynthesis pathway of carbohydrates, nucleic acids encoding a protein from the biosynthesis pathway of aromatic compound, nucleic acids encoding a protein from the biosynthesis pathway of vitamins, nucleic acids encoding a protein from the biosynthesis pathway of carotenoids, in particular ketocarotenoids, nucleic acids encoding a protein from the biosynthesis pathway of cofactors and nucleic acids encoding a protein from the biosynthesis pathway of enzymes.

Preferred fine chemicals or biosynthetic products which can be produced in plants of the genus Tagetes, in particular in petals of the flowers of the plants of the genus Tagetes, are carotenoids, such as, for example, phytoene, lycopene, lutein, zeaxanthin, astaxanthin, canthaxanthin, echinenone, 3-hydroxyechinenone, 3'-hydroxyechinenone, adonirubin, violaxanthin and adonixanthin.

Particularly preferred carotenoids are ketocarotenoids, such as, for example, astaxanthin, canthaxanthin, echinenone, 3-hydroxyechinenone, 3'-hydroxyechinenone, adonirubin and adonixanthin.

Very particularly preferred genes which are expressed using the promoters according to the invention in plants of the genus Tagetes are accordingly genes which encode proteins from the biosynthesis pathway of carotenoids.

Genes selected from the group consisting of nucleic acids encoding a beta-hydroxylase, nucleic acids encoding a beta-cyclase, nucleic acids encoding an epsilon-cyclase, nucleic acids encoding a zeaxanthin-epoxidase, nucleic acids encoding a antheraxanthin-epoxidase, nucleic acids encoding a neoxanthin-synthase, nucleic acids encoding an HMG-CoA reductase, nucleic acids encoding an (E)-4-hydroxy-3-methylbut-2-enyl-diphosphate reductase, nucleic acids encoding a 1-deoxy-D-xylose-5-phosphate synthase, nucleic acids encoding a 1-deoxy-D-xylose-5-phosphate reductoisomerase, nucleic acids encoding an isopentenyl diphosphate delta-isomerase, nucleic acids encoding a geranyl diphosphate synthase, nucleic acids encoding a farnesyl diphosphate synthase, nucleic acids encoding a geranylgeranyl diphosphate synthase, nucleic acids encoding a phytoene synthase, nucleic acids encoding a phytoene desaturase, (phytoenedehydrogenase), nucleic acids encoding a prephytoene synthase, nucleic acids encoding a zeta-carotene desaturase, nucleic acids encoding a crtISO protein, nucleic acids encoding a 4-diphosphocytidyl-2-C-methyl-D-erythritol synthase, nucleic acids encoding a 4-diphosphocytidyl-2-C-methyl-D-erythritol kinase, nucleic acids encoding a 2-Methyl-D-erythritol cyclophosphate-synthase, nucleic acids encoding a Hydroxymethylbutenyldiphosphate-synthase, nucleic acids encoding an FtsZ protein and nucleic acids encoding a MinD protein are particularly preferred.

A beta-cyclase is understood as meaning a protein which has the enzymatic activity to convert a terminal, linear residue of lycopene to a beta-ionone ring.

In particular, a beta-cyclase is understood as meaning a protein which has the enzymatic activity to convert gamma-carotene to beta-carotene.

Examples of beta-cyclase genes are nucleic acids encoding a beta-cyclase from tomato (Accession X86452), and beta-cyclases of the following accession numbers:

| | |
|---|---|
| S66350 | lycopene beta-cyclase (EC 5.5.1.-) - tomato |
| CAA60119 | lycopene synthase [Capsicum annuum] |
| S66349 | lycopene beta-cyclase (EC 5.5.1.-) - common tobacco |
| CAA57386 | lycopene cyclase [Nicotiana tabacum] |
| AAM21152 | lycopene beta-cyclase [Citrus sinensis] |
| AAD38049 | lycopene cyclase [Citrus x paradisi] |
| AAN86060 | lycopene cyclase [Citrus unshiu] |
| AAF44700 | lycopene beta-cyclase [Citrus sinensis] |
| AAK07430 | lycopene beta-cyclase [Adonis palaestina] |
| AAG10429 | beta cyclase [Tagetes erecta] |
| AAA81880 | lycopene cyclase |
| AAB53337 | Lycopene beta cyclase |
| AAL92175 | beta-lycopene cyclase [Sandersonia aurantiaca] |
| CAA67331 | lycopene cyclase [Narcissus pseudonarcissus] |
| AAM45381 | beta cyclase [Tagetes erecta] |
| AA018661 | lycopene beta-cyclase [Zea mays] |
| AAG21133 | chromoplast-specific lycopene beta-cyclase [Lycopersicon esculentum] |
| AAF18989 | lycopene beta-cyclase [Daucus carota] |
| ZP_001140 | hypothetical protein [Prochlorococcus marinus str. MIT9313] |
| ZP_001050 | hypothetical protein [Prochlorococcus marinus subsp. pastoris str. CCMP1378] |
| ZP_001046 | hypothetical protein [Prochlorococcus marinus subsp. pastoris str. CCMP1378] |
| ZP_001134 | hypothetical protein [Prochlorococcus marinus str. MIT9313] |
| ZP_001150 | hypothetical protein [Synechococcus sp. WH 8102] |
| AAF10377 | lycopene cyclase [Deinococcus radiodurans] |
| BAA29250 | 393aa long hypothetical protein [Pyrococcus horikoshii] |
| BAC77673 | lycopene beta-monocyclase [marine bacterium P99-3] |
| AAL01999 | lycopene cyclase [Xanthobacter sp. Py2] |
| ZP_000190 | hypothetical protein [Chloroflexus aurantiacus] |
| ZP_000941 | hypothetical protein [Novosphingobium aromaticivorans] |
| AAF78200 | lycopene cyclase [Bradyrhizobium sp. ORS278] |
| BAB79602 | crtY [Pantoea agglomerans pv. milletiae] |
| CAA64855 | lycopene cyclase [Streptomyces griseus] |
| AAA21262 | dycopene cyclase [Pantoea agglomerans] |
| C37802 | crtY protein - Erwinia uredovora |
| BAB79602 | crtY [Pantoea agglomerans pv. milletiae] |
| AAA64980 | lycopene cyclase [Pantoea agglomerans] |
| AAC44851 | lycopene cyclase |
| BAA09593 | Lycopene cyclase [Paracoccus sp. MBIC1143] |
| ZP_000941 | hypothetical protein [Novosphingobium aromaticivorans] |
| CAB56061 | lycopene beta-cyclase [Paracoccus marcusii] |
| BAA20275 | lycopene cyclase [Erythrobacter longus] |
| ZP_000570 | hypothetical protein [Thermobifida fusca] |
| ZP_000190 | hypothetical protein [Chloroflexus aurantiacus] |
| AAK07430 | lycopene beta-cyclase [Adonis palaestina] |
| CAA67331 | lycopene cyclase [Narcissus pseudonarcissus] |
| AAB53337 | Lycopene beta cyclase |
| BAC77673 | lycopene beta-monocyclase [marine bacterium P99-3] |

A particularly preferred beta-cyclase is furthermore the chromoplast-specific beta-cyclase from tomato (AAG21133)

A hydroxylase is understood as meaning a protein which has the enzymatic activity to introduce a hydroxyl group on the optionally substituted, β-ionone ring of carotenoids. In particular, a hydroxylase is understood as meaning a protein which has the enzymatic activity to convert -carotene to zeaxanthin or canthaxanthin to astaxanthin.

Examples of a hydroxylase gene are:
a nucleic acid encoding a hydroxylase from Haematococcus pluvialis, Accession AX038729, WO 0061764); (nucleic acid: SEQ ID NO: 49, protein: SEQ ID NO: 50),
   and hydroxylases of the following accession numbers:
   |emb|CAB55626.1, CAA70427.1, CAA70888.1, CAB55625.1, AF499108_1, AF315289_1, AF296158_1, AAC49443.1, NP_194300.1, NP_200070.1, AAG10430.1, CAC06712.1, AAM88619.1, CAC95130.1, AAL80006.1, AF162276_1, AAO53295.1, AAN85601.1, CRTZ_ERWHE, CRTZ_PANAN, BAB79605.1, CRTZ_ALCSP, CRTZ_AGRAU, CAB56060.1, ZP_00094836.1, AAC44852.1, BAC77670.1, NP_745389.1, NP_344225.1, NP_849490.1, ZP_00087019.1, NP_503072.1, NP_852012.1, NP_115929.1, ZP_00013255.1

A particularly preferred hydroxylase is furthermore the hydroxylase from tomato (Accession Y14810, CrtR-b2) (nucleic acid: SEQ ID NO: 51; protein: SEQ ID NO. 52).

An HMG-CoA reductase is understood as meaning a protein which has the enzymatic activity to convert 3-hydroxy-3-methyl-glutarylcoenzyme A to mevalonate.

An (E)-4-hydroxy-3-methylbut-2-enyl-diphosphate reductase is understood as meaning a protein which has the enzymatic activity to convert (E)-4-hydroxy-3-methylbut-2-enyl diphosphate to isopentenyl diphosphate and dimethylallyl diphosphates.

A 1-deoxy-D-xylose-5-phosphate synthase is understood as meaning a protein which has the enzymatic activity to convert hydroxyethyl-ThPP and glyceraldehyde 3-phosphate to 1-deoxy-D-xylose 5-phosphate.

A 1-deoxy-D-xylose-5 phosphate reductoisomerase is understood as meaning a protein which has the enzymatic activity to convert 1-deoxy-D-xylose 5-phosphate to 2-C-methyl-D-erythritol 4-phosphate.

An isopentenyl diphosphate delta-isomerase is understood as meaning a protein which has the enzymatic activity to convert isopentenyl diphosphate to dimethylallyl phosphate.

A geranyl diphosphate synthase is understood as meaning a protein which has the enzymatic activity to convert isopentenyl diphosphate and dimethylallyl phosphate to geranyl diphosphate.

A farnesyl diphosphate synthase is understood as meaning a protein which has the enzymatic activity to convert sequentially 2 molecules of isopentenyl diphosphate using dimethylallyl diphosphate and the resulting geranyl diphosphate to farnesyl diphosphate.

A geranylgeranyl diphosphate synthase is understood as meaning a protein which has the enzymatic activity to convert farnesyl diphosphate and isopentenyl diphosphate to geranylgeranyl diphosphate.

A phytoene synthase is understood as meaning a protein which has the enzymatic activity to convert geranylgeranyl diphosphate to phytoene.

A phytoene desaturase is understood as meaning a protein which has the enzymatic activity to convert phytoene to phytofluene and/or phytofluene to zeta-carotene.

A zeta-carotene desaturase is understood as meaning a protein which has the enzymatic activity to convert zeta-carotene to neurosporin and/or neurosporin to lycopene.

A crtlSO protein is understood as meaning a protein which has the enzymatic activity to convert 7,9,7',9'-tetra-cis-lycopene to all-trans-lycopene.

An FtsZ protein is understood as meaning a protein which has a cell division and plastidic division-promoting action and has homologies to tubulin proteins.

A MinD protein is understood as meaning a protein which has a multifunctional role in cell division. It is a membrane-associated ATPase and can show within the cell an oscillating motion from pole to pole.

Examples of HMG-CoA reductase genes are:
a nucleic acid encoding an HMG-CoA reductase from Arabidopsis thaliana, Accession NM_106299;
and further HMG-CoA reductase genes from other organisms having the following accession numbers:
   P54961, P54870, P54868, P54869, 002734, P22791, P54873, P54871, P23228, P13704, P54872, Q01581, P17425, P54874, P54839, P14891, P34135, 064966, P29057, P48019, P48020, P12683, P43256, Q9XEL8, P34136, O64967, P29058, P48022, Q41437, P12684, Q00583, Q9XHL5, Q41438, Q9YAS4, 076819, 028538, Q9Y7D2, P54960, 051628, P48021, Q03163, P00347, P14773, Q12577, Q59468, P04035, O24594, P09610, Q58116, 026662, Q01237, Q01559, Q12649, O74164, 059469, P51639, Q10283, 008424, P20715, P13703, P13702, Q96UG4, Q8SQZ9, 015888, Q9TUM4, P93514, Q39628, P93081, P93080, Q944T9, Q40148, Q84MM0, Q84LS3, Q9Z9N4, Q9KLM0

Examples of (E)-4-hydroxy-3-methylbut-2-enyl diphosphate reductase genes are:
A nucleic acid encoding an (E)-4-hydroxy-3-methylbut-2-enyl diphosphate reductase from Arabidopsis thaliana (lytB/ISPH), ACCESSION AY168881, and further (E)-4-hydroxy-3-methylbut-2-enyl-diphosphate reductase genes from other organisms having the following accession numbers:
   T04781, AF270978_1, NP_485028.1, NP_442089.1, NP_681832.1, ZP_00110421.1, ZP_00071594.1, ZP_00114706.1, ISPH_SYNY3, ZP_00114087.1, ZP_00104269.1, AF398145_1, AF398146_1, AAD55762.1, AF514843_1, NP_622970.1, NP_348471.1, NP_562001.1, NP_223698.1, NP_781941.1, ZP_00080042.1, NP_859669.1, NP_214191.1, ZP_00086191.1, ISPH_VIBCH, NP_230334.1, NP_742768.1, NP_302306.1, ISPH_MYCLE, NP_602581.1, ZP_00026966.1, NP_520563.1, NP_253247.1, NP_282047.1, ZP_00038210.1, ZP_00064913.1, CAA61555.1, ZP_00125365.1, ISPH_ACICA, EAA24703.1, ZP_00013067.1, ZP_00029164.1, NP_790656.1, NP_217899.1, NP_641592.1, NP_636532.1, NP_719076.1, NP_660497.1, NP_422155.1, NP_715446.1, ZP_00090692.1, NP_759496.1, ISPH_BURPS, ZP_00129657.1, NP_215626.1, NP_335584.1, ZP_00135016.1, NP_789585.1, NP_787770.1, NP_769647.1, ZP_00043336.1, NP_242248.1, ZP_00008555.1, NP_246603.1, ZP_00030951.1, NP_670994.1, NP_404120.1, NP_540376.1, NP_733653.1, NP_697503.1, NP_840730.1, NP_274828.1, NP_796916.1, ZP_00123390.1, NP_824386.1, NP_737689.1, ZP_00021222.1, NP_757521.1, NP_390395.1, ZP_00133322.1, CAD76178.1, NP_600249.1, NP_454660.1, NP_712601.1, NP_385018.1, NP_751989.1

Examples of 1-deoxy-D-xylose-5-phosphate synthase genes are:
A nucleic acid encoding a 1-deoxy-D-xylose-5-phosphate synthase from Lycopersicon esculentum, ACCESSION #AF143812 and further 1-deoxy-D-xylose-5-phosphate synthase genes from other organisms having the following accession numbers:
   AF143812_1, DXS_CAPAN, CAD22530.1, AF182286_1, NP_193291.1, T52289, AAC49368.1, AAP14353.1, D71420, DXS_ORYSA, AF443590_1, BAB02345.1, CAA09804.2, NP_850620.1, CAD22155.2, AAM65798.1, NP_566686.1, CAD22531.1, AAC33513.1, CAC08458.1, AAG10432.1, T08140, AAP14354.1, AF428463_1, ZP_00010537.1, NP_769291.1, AAK59424.1, NP_107784.1, NP_697464.1, NP_540415.1, NP_196699.1, NP_384986.1, ZP_00096461.1, ZP_00013656.1, NP_353769.1, BAA83576.1, ZP_00005919.1, ZP_00006273.1, NP_420871.1, AAM48660.1, DXS_RHOCA, ZP_00045608.1, ZP_00031686.1, NP_841218.1, ZP_00022174.1, ZP_00086851.1, NP_742690.1, NP_520342.1, ZP_00082120.1, NP_790545.1, ZP_00125266.1, CAC17468.1, NP_252733.1, ZP_00092466.1, NP_439591.1, NP_414954.1, NP_752465.1, NP_622918.1, NP_286162.1, NP_836085.1, NP_706308.1, ZP_00081148.1, NP_797065.1, NP_213598.1, NP_245469.1, ZP_00075029.1, NP_455016.1, NP_230536.1, NP_459417.1, NP_274863.1, NP_283402.1, NP_759318.1, NP_406652.1, DXS_SYNLE, DXS_SYNP7, NP_440409.1, ZP_00067331.1, ZP_00122853.1, NP_717142.1, ZP_00104889.1, NP_243645.1, NP_681412.1, DXS_SYNEL, NP_637787.1, DXS_CHLTE, ZP_00129863.1, NP_661241.1, DXS_XANCP, NP_470738.1, NP_484643.1, ZP_00108360.1, NP_833890.1, NP_846629.1, NP_658213.1, NP_642879.1, ZP_00039479.1, ZP_00060584.1, ZP_00041364.1, ZP_00117779.1, NP_299528.1

Examples of 1-deoxy-D-xylose-5-phosphate reductoisomerase genes are:
A nucleic acid encoding a 1-deoxy-D-xylose-5-phosphate reductoisomerase from Arabidopsis thaliana, ACCESSION #AF148852,
   and further 1-deoxy-D-xylose-5-phosphate reductoisomerase genes from other organisms having the following accession numbers:

AF148852, AY084775, AY054682, AY050802, AY045634, AY081453, AY091405, AY098952, AJ242588, AB009053, AY202991, NP_201085.1, T52570, AF331705_1, BAB16915.1, AF367205_1, AF250235_1, CAC03581.1, CAD22156.1, AF182287_1, OXR_MENPI, ZP_00071219.1, NP_488391.1, ZP_00111307.1, DXR_SYNLE, AAP56260.1, NP_681831.1, NP_442113.1, ZP_00115071.1, ZP_00105106.1, ZP_00113484.1, NP_833540.1, NP_657789.1, NP_661031.1, DXR_BACHD, NP_833080.1, NP_845693.1, NP_562610.1, NP_623020.1, NP_810915.1, NP_243287.1, ZP_00118743.1, NP_464842.1, NP_470690.1, ZP_00082201.1, NP_781898.1, ZP_00123667.1, NP_348420.1, NP_604221.1, ZP_00053349.1, ZP_00064941.1, NP_246927.1, NP_389537.1, ZP_00102576.1, NP_519531.1, AF124757_19, DXR_ZYMMO, NP_713472.1, NP_459225.1, NP_454827.1, ZP_00045738.1, NP_743754.1, DXR_PSEPK, ZP_00130352.1, NP_702530.1, NP_841744.1, NP_438967.1, AF514841_1, NP_706118.1, ZP_00125845.1, NP_404661.1, NP_285867.1, NP_240064.1, NP_414715.1, ZP_00094058.1, NP_791365.1, ZP_00012448.1, ZP_00015132.1, ZP_00091545.1, NP_629822.1, NP_771495.1, NP_798691.1, NP_231885.1, NP_252340.1, ZP_00022353.1, NP_355549.1, NP_420724.1, ZP_00085169.1, EAA17616.1, NP_273242.1, NP_219574.1, NP_387094.1, NP_296721.1, ZP_00004209.1, NP_823739.1, NP_282934.1, BAA77848.1, NP_660577.1, NP_760741.1, NP_641750.1, NP_636741.1, NP_829309.1, NP_298338.1, NP_444964.1, NP_717246.1, NP_224545.1, ZP_00038451.1, DXR_KITGR, NP_778563.1.

Examples of isopentenyl diphosphate delta-isomerase genes are:
A nucleic acid encoding an isopentenyl diphosphate delta-isomerase from Adonis palaestina clone ApIPI28, (ipiAa1), ACCESSION #AF188060, published by Cunningham, F.X. Jr. and Gantt, E.: Identification of multi-gene families encoding isopentenyl diphosphate isomerase in plants by heterologous complementation in Escherichia coli, Plant Cell Physiol. 41 (1), 119-123 (2000) and further isopentenyl diphosphate -isomerase genes from other organisms having the following accession numbers:
   Q38929, 048964, Q39472, Q13907, 035586, P58044, 042641, 035760, Q10132, P15496, Q9YB30, Q8YNH4, Q42553, 027997, P50740, 051627, 048965, Q8KFR5,
   Q39471, Q39664, Q9RVE2, Q01335, Q9HHE4, Q9BXS1, Q9KWF6, Q9CIF5, Q88WB6, Q92BX2, Q8Y7A5, Q8TT35 Q9KK75, Q8NN99, Q8XD58, Q8FE75, Q46822, Q9HP40, P72002, P26173, Q9Z5D3, Q8Z3X9, Q8ZM82, Q9X7Q6, 013504, Q9HFW8, Q8NJL9, Q9UUQ1, Q9NH02, Q9M6K9, Q9M6K5, Q9FXR6, 081691, Q9S7C4, Q8S3L8, Q9M592, Q9M6K3, Q9M6K7, Q9FV48, Q9LLB6, Q9AVJ1, Q9AVG8, Q9M6K6, Q9AVJ5, Q9M6K2, Q9AYS5, Q9M6K8, Q9AVG7, Q8S3L7, Q8W250, Q941E1, Q9AV18, Q9AYS6, Q9SAY0, Q9M6K4, Q8GVZ0, Q84RZ8, Q8KZ12, Q8KZ66, Q8FND7, Q88QC9, Q8BFZ6, BAC26382, CAD94476.

Examples of geranyl diphosphate synthase genes are:
A nucleic acid encoding a geranyl diphosphate synthase from Arabidopsis thaliana, ACCESSION #Y17376, Bouvier, F., Suire, C., d'Harlingue, A., Backhaus, R.A. and Camara, B.; Molecular cloning of geranyl diphosphate synthase and compartmentation of monoterpene synthesis in plant cells, Plant J. 24 (2), 241-252 (2000) and further geranyl diphosphate synthase genes from other organisms having the following accession numbers:
   Q9FT89, Q8LKJ2, Q9FSW8, Q8LKJ3, Q9SBR3, Q9SBR4, Q9FET8, Q8LKJ1, Q84LG1, Q9JK86

Examples of farnesyl diphosphate synthase genes are:
A nucleic acid encoding a farnesyl diphosphate synthase from Arabidopsis thaliana (FPS1), ACCESSION #U80605, published by Cunillera, N., Arro, M., Delourme, D., Karst, F., Boronat, A. and Ferrer, A.: Arabidopsis thaliana contains two differentially expressed farnesyl-diphosphate synthase genes, J. Biol. Chem. 271 (13), 7774-7780 (1996),and further farnesyl diphosphate synthase genes from other organisms having the following accession numbers :
   P53799, P37268, Q02769, Q09152, P49351, 024241, Q43315, P49352, 024242, P49350, P08836, P14324, P49349, P08524, 066952, Q08291, P54383, Q45220, P57537, Q8K9A0, P22939, P45204, 066126, P55539, Q9SWH9, Q9AV17, Q9FRX2, Q9AYS7, Q94IE8, Q9FXR9, Q9ZWF6, Q9FXR8, Q9AR37, O50009, Q94IE9, Q8RVK7, Q8RVQ7, 004882, Q93RA8, Q93RB0, Q93RB4, Q93RB5, Q93RB3, Q93RB1, Q93RB2, Q920E5.

Examples of geranylgeranyl diphosphate synthase genes are:
A nucleic acid encoding a geranylgeranyl diphosphate synthase from Sinaps alba, ACCESSION #X98795, published by Bonk, M., Hoffmann, B., Von Lintig, J., Schledz, M., Al-Babili, S., Hobeika, E., Kleinig, H. and Beyer, P.: Chloroplast import of four carotenoid biosynthetic enzymes in vitro reveals differential fates prior to membrane binding and oligomeric assembly, Eur. J. Biochem. 247 (3), 942-950 (1997),
   and further geranylgeranyl diphosphate synthase genes from other organisms having the following accession numbers:
   P22873, P34802, P56966, P80042, Q42698, Q92236, 095749, Q9WTN0, Q50727, P24322, P39464, Q9FXR3, Q9AYN2, Q9FXR2, Q9AVG6, Q9FRW4, Q9SXZ5, Q9AVJ7, Q9AYN1, Q9AVJ4, Q9FXR7, Q8LSC5, Q9AVJ6, Q8LSC4, Q9AVJ3, Q9SSU0, Q9SXZ6, Q9SST9, Q9AVJ0, Q9AVI9, Q9FRW3, Q9FXR5, Q94IF0, Q9FRX1, Q9K567, Q93RA9, Q93QX8, CAD95619, EAA31459

Examples of phytoene synthase genes are:
A nucleic acid encoding a phytoene synthase from Erwinia uredovora, ACCESSION # D90087; published by Misawa, N., Nakagawa, M., Kobayashi, K., Yamano, S., Izawa, Y., Nakamura, K. and Harashima, K.: Elucidation of the Erwinia uredovora carotenoid biosynthetic pathway by functional analysis of gene products expressed in Escherichia coli; J. Bacteriol. 172 (12), 6704-6712 (1990), and further phytoene synthase genes from other organisms having the following accession numbers:
   CAB39693, BAC69364, AAF10440, CAA45350, BAA20384, AAM72615, BAC09112, CAA48922, P_001091, CAB84588, AAF41518, CAA48155, AAD38051, AAF33237, AAG10427, AAA34187, BAB73532, CAC19567, AAM62787, CAA55391, AAB65697, AAM45379, CAC27383, AAA32836, AAK07735, BAA84763, P_000205, AAB60314, P_001163, P_000718, AAB71428, AAA34153, AAK07734, CAA42969, CAD76176, CAA68575, P_000130, P_001142, CAA47625, CAA85775, BAC14416, CAA79957, BAC76563, P_000242, P_000551, AAL02001, AAK15621, CAB94795, AAA91951, P_000448

Examples of phytoene desaturase genes are:
A nucleic acid encoding a phytoene desaturase from Erwinia uredovora, ACCESSION # D90087; published by Misawa, N., Nakagawa, M., Kobayashi, K., Yamano, S., Izawa, Y., Nakamura, K. and Harashima, K.: Elucidation of the Erwinia uredovora carotenoid biosynthetic pathway by functional analysis of gene products expressed in Escherichia coli; J. Bacteriol. 172 (12), 6704-6712 (1990), (nucleic acid: SEQ ID NO: 71, protein: SEQ ID NO: 72),
   and further phytoene desaturase genes from other organisms having the following accession numbers:
   AAL15300, A39597, CAA42573, AAK51545, BAB08179, CAA48195, BAB82461, AAK92625, CAA55392, AAG10426, AAD02489, AAO24235, AAC12846, AAA99519, AAL38046, CAA60479, CAA75094, ZP_001041, ZP_001163, CAA39004, CAA44452, ZP_001142, ZP_000718, BAB82462, AAM45380, CAB56040, ZP_001091, BAC09113, AAP79175, AAL80005, AAM72642, AAM72043, ZP_000745, ZP_001141, BAC07889, CAD55814, ZP_001041, CAD27442, CAE00192, ZP_001163, ZP_000197, BAA18400, AAG10425, ZP_001119, AAF13698, 2121278A, AAB35386, AAD02462, BAB68552, CAC85667, AAK51557, CAA12062, AAG51402, AAM63349, AAF85796, BAB74081, AAA91161, CAB56041, AAC48983, AAG14399, CAB65434, BAB73487, ZP_001117, ZP_000448, CAB39695, CAD76175, BAC69363, BAA17934, ZP_000171, AAF65586, ZP_000748, BAC07074, ZP_001133, CAA64853, BAB74484, ZP_001156, AAF23289, AAG28703, AAP09348, AAM71569, BAB69140, ZP_000130, AAF41516, AAG18866, CAD95940, NP_656310, AAG10645, ZP_000276, ZP_000192, ZP_000186, AAM94364, EAA31371, ZP_000612, BAC75676, AAF65582

Examples of zeta-carotene desaturase genes are:
A nucleic acid encoding a zeta-carotene desaturase from Narcissus pseudonarcissus, ACCESSION #AJ224683, published by Al-Babili, S., Oelschlegel, J. and Beyer, P.: A cDNA encoding for beta carotene desaturase (Accession No.AJ224683) from Narcissus pseudonarcissus L.. (PGR98-103), Plant Physiol. 117, 719-719 (1998), and further zeta-carotene desaturase genes from other organisms having the following accession numbers:
   Q9R6X4, Q38893, Q9SMJ3, Q9SE20, Q9ZTP4, 049901, P74306, Q9FV46, Q9RCT2, ZDS_NARPS, BAB68552.1, CAC85667.1, AF372617_1, ZDS_TARER, CAD55814.1, CAD27442.1, 2121278A, ZDS_CAPAN, ZDS_LYCES, NP_187138.1, AAM63349.1, ZDS_ARATH, AAA91161.1, ZDS_MAIZE, AAG14399.1, NP_441720.1, NP_486422.1, ZP_00111920.1, CAB56041.1, ZP_00074512.1, ZP_00116357.1, NP_681127.1, ZP_00114185.1, ZP_00104126.1, CAB65434.1, NP_662300.1

Examples of crtISO genes are:
A nucleic acid encoding a crtISO from Lycopersicon esculentum; ACCESSION #AF416727, published by Isaacson, T., Ronen, G., Zamir, D. and Hirschberg, J.: Cloning of tangerine from tomato reveals a carotenoid isomerase essential for the production of beta-carotene and xanthophylls in plants; Plant Cell 14 (2), 333-342 (2002), and further crtISO genes from other organisms having the following accession numbers:
   AAM53952

Examples of FtsZ genes are:
A nucleic acid encoding an FtsZ from Tagetes erecta, ACCESSION #AF251346, published by Moehs, C.P., Tian, L., Osteryoung, K.W. and Dellapenna, D.: Analysis of carotenoid biosynthetic gene expression during marigold petal development Plant Mol. Biol. 45 (3), 281-293 (2001), and further FtsZ genes from other organisms having the following accession numbers:
   CAB89286.1, AF205858_1, NP_200339.1, CAB89287.1, CAB41987.1, AAA82068.1, T06774,AF383876_1, BAC57986.1, CAD22047.1, BAB91150.1, ZP_00072546.1, NP_440816.1, T51092, NP_683172.1, BAA85116.1, NP_487898.1, JC4289, BAA82871.1, NP_781763.1, BAC57987.1, ZP_00111461.1, T51088, NP_190843.1, ZP_00060035.1, NP_846285.1, AAL07180.1, NP_243424.1, NP_833626.1, AAN04561.1, AAN04557.1, CAD22048.1, T51089, NP_692394.1, NP_623237.1, NP_565839.1, T51090, CAA07676.1, NP_113397.1, T51087, CAC44257.1, E84778, ZP_00105267.1, BAA82091.1, ZP_00112790.1, BAA96782.1, NP_348319.1, NP_471472.1, ZP_00115870.1, NP_465556.1, NP_389412.1, BAA82090.1, NP_562681.1, AAM22891.1, NP_371710.1, NP_764416.1, CAB95028.1, FTSZ_STRGR, AF120117_1, NP_827300.1, JE0282, NP_626341.1, AAC45639.1,
   NP_785689.1, NP_336679.1, NP_738660.1, ZP_00057764.1, AAC32265.1, NP_814733.1, FTSZ_MYCKA, NP_216666.1, CAA75616.1, NP_301700.1, NP_601357.1, ZP_00046269.1, CAA70158.1, ZP_00037834.1, NP_268026.1, FTSZ_ENTHR, NP_787643.1, NP_346105.1, AAC32264.1, JC5548, AAC95440.1, NP_710793.1, NP_687509.1, NP_269594.1, AAC32266.1, NP_720988.1, NP_657875.1, ZP_00094865.1, ZP_00080499.1, ZP_00043589.1, JC7087, NP_660559.1, AAC46069.1, AF179611_14, AAC44223.1, NP_404201.1.

Examples of MinD genes are:
A nucleic acid encoding a MinD from Tagetes erecta, ACCESSION #AF251019, published by Moehs, C.P., Tian, L., Osteryoung, K.W. and Dellapenna, D.: Analysis of carotenoid biosynthetic gene expression during marigold petal development; Plant Mol. Biol. 45 (3), 281-293 (2001), (nucleic acid: SEQ ID NO: 79, protein: SEQ ID NO: 80),
   and further MinD genes having the following accession numbers:
   NP_197790.1, BAA90628.1, NP_038435.1, NP_045875.1, AAN33031.1, NP_050910.1, CAB53105.1, NP_050687.1, NP_682807.1, NP_487496.1, ZP_00111708.1, ZP_00071109.1, NP_442592.1, NP_603083.1, NP_782631.1, ZP_00097367.1, ZP_00104319.1, NP_294476.1, NP_622555.1, NP_563054.1, NP_347881.1, ZP_00113908.1, NP_834154.1, NP_658480.1. ZP_00059858.1, NP_470915.1, NP_243893.1, NP_465069.1, ZP_00116155.1, NP_390677.1, NP_692970.1, NP_298610.1, NP_207129.1, ZP_00038874.1, NP_778791.1, NP_223033.1, NP_641561.1, NP_636499.1, ZP_00088714.1, NP_213595.1, NP_743889.1, NP_231594.1, ZP_00085067.1, NP_797252.1, ZP_00136593.1, NP_251934.1, NP_405629.1, NP_759144.1, ZP_00102939.1, NP_793645.1, NP_699517.1, NP_460771.1, NP_860754.1, NP_456322.1, NP_718163.1, NP_229666.1, NP_357356.1, NP_541904.1, NP_287414.1, NP_660660.1, ZP_00128273.1, NP_103411.1, NP_785789.1, NP_715361.1, AF149810_1, NP_841854.1, NP_437893.1, ZP_00022726.1, EAA24844.1, ZP_00029547.1, NP_521484.1, NP_240148.1, NP_770852.1, AF345908_2, NP_777923.1, ZP_00048879.1, NP_579340.1, NP_143455.1, NP_126254.1, NP_142573.1, NP_613505.1, NP_127112.1, NP_712786.1, NP_578214.1, NP_069530.1, NP_247526.1, AAA85593.1, NP_212403.1, NP_782258.1, ZP_00058694.1, NP_247137.1, NP_219149.1, NP_276946.1, NP_614522.1, ZP_00019288.1, CAD78330.1

The invention further relates to a genetically modified plant of the genus Tagetes, the genetic modification leading to an increasing or causing of the expression rate of at least one gene in comparison with the wild-type and being due to the regulation of the expression of this gene in the plant by the polynucleotide sequences according to the invention.

As mentioned above, "expression activity" is understood according to the invention as meaning the amounts of protein formed in a certain time by the promoter, that is the expression rate.

"Specific expression activity" is understood according to the invention as meaning the amount of protein per promoter formed in a certain time by the promoter.

In a "caused expression activity" or "caused expression rate" in relation to a gene in comparison with the wild-type, in comparison with the wild-type the formation of a protein is thus caused which was not present in the wild-type of the plant of the genus Tagetes.

For example, wild-type plants of the genus Tagetes have no ketolase gene. The regulation of the expression of the ketolase gene in the plant by the promoters according to the invention thus leads to a causing of the expression rate.

In an "increased expression activity" or "increased expression rate" in relation to a gene in comparison with the wild-type, in comparison with the wild-type the amount of protein formed in a certain time is thus increased in the plant of the genus Tagetes.

For example, wild-type plants of the genus Tagetes have a hydroxylase gene. The regulation of the expression of the hydroxylase gene in the plant by the promoters according to the invention thus leads to an increasing of the expression rate.

In a preferred embodiment of the genetically modified plants of the genus Tagetes according to the invention, the regulation of the expression of ketolase genes according to the invention is achieved by means of the promoters according to the invention, in that one or more nucleic acid constructs comprising at least one promoter according to the invention and, functionally linked, one or more ketolase genes to be expressed are inserted into the plant.

In a preferred embodiment one or more nucleic acid constructs comprising at least one promoter according to the invention and, functionally linked, one ketolase gene according to the invention and more genes to be expressed are inserted into the plant. The integration of the nucleic acid constructs into the plant of the genus Tagetes can in this case take place intrachromosomally or extrachromosomally.

Preferred promoters according to the invention and preferred genes to be expressed (effect genes) are described above.

By way of example, the production of the genetically modified plants of the genus Tagetes having an increased or caused expression rate of an effect gene is described below.

The transformation can be carried out individually or by means of multiple constructs in the combinations of genetic modifications.

The production of the transgenic plants is preferably carried out by transformation of the starting plants, using a nucleic acid construct which comprises at least one of the promoters according to the invention described above, which are functionally linked to an effect gene to be expressed and, if appropriate, to further regulation signals.

These nucleic acid constructs, in which the promoters according to the invention and effect genes are functionally linked, are also called expression cassettes below.

The expression cassettes can comprise further regulation signals, that is regulative nucleic acid sequences, which control the expression of the effect genes in the host cell. According to a preferred embodiment, an expression cassette comprises upstream, i.e. at the 5'-end of the coding sequence, at least one promoter according to the invention and downstream, i.e. at the 3'-end, a polyadenylation signal and, if appropriate, further regulatory elements which are operatively linked with the intermediate coding sequence of the effect gene for at least one of the genes described above.

An operative linkage is understood as meaning the sequential arrangement of promoter, coding sequence, terminator and, if appropriate, further regulative elements in such a way that each of the regulative elements can fulfill its function in the expression of the coding sequence as intended.

By way of example, the preferred nucleic acid constructs, expression cassettes and vectors for plants and processes for the production of transgenic plants, and the transgenic plants of the genus Tagetes themselves are described below.

The sequences which are preferred, but not restricted thereto, for the operative linkage are targeting sequences for the guaranteeing of the subcellular location in the apoplast, in the vacuoles, in plastids, in the mitochondrium, in the endoplasmatic reticulum (ER), in the cell nucleus, in elaioplasts or other compartments and translation enhancers such as the 5'-guiding sequence of the tobacco mosaic virus (Gallie et al., Nucl. Acids Res. 15 (1987), 8693-8711).

The production of an expression cassette is preferably carried out by fusion of at least one promoter according to the invention with at least one gene, preferably with one of the effect genes described above, and preferably a nucleic acid inserted between promoter and nucleic acid sequence, which codes for a plastid-specific transit peptide, and a polyadenylation signal according to customary recombination and cloning techniques, such as are described, for example, in T. Maniatis, E.F. Fritsch and J. Sambrook, Molecular Cloning: A Laboratory Manual, Cold Spring Harbor Laboratory, Cold Spring Harbor, NY (1989) and also in T.J. Silhavy, M.L. Berman and L.W. Enquist, Experiments with Gene Fusions, Cold Spring Harbor Laboratory, Cold Spring Harbor, NY (1984) and in Ausubel, F.M. et al., Current Protocols in Molecular Biology, Greene Publishing Assoc, and Wiley-Interscience (1987).

The preferably inserted nucleic acids, encoding a plastidic transit peptide, guarantee location in plastids and in particular in chromoplasts.

Expression cassettes can also be used whose nucleic acid sequence codes for an effect gene-product fusion protein, a part of the fusion protein being a transit peptide which controls the translocation of the polypeptide. Specific transit peptides, which are removed enzymatically from the effect gene product part after translocation of the effect genes to the chromoplasts, are preferred for the chromoplasts.

The transit peptide which is derived from the plastidic Nicotiana tabacum transketolase or another transit peptide (e.g. the transit peptide of the small subunit of the Rubisco (rbcS) or of the ferredoxin NADP oxidoreductase and the isopentenyl pyrophosphate isomerase-2 or its functional equivalent) is particularly preferred.

Further examples of a plastidic transit peptide are the transit peptide of the plastidic isopentenyl pyrophosphate isomerase-2 (IPP-2) from Arabisopsis thaliana and the transit peptide of the small subunit of the ribulose bisphosphate carboxylase (rbcS) from pea (Guerineau, F, Woolston, S, Brooks, L, Mullineaux, P (1988) An expression cassette for targeting foreign proteins into the chloroplasts. Nucl. Acids Res. 16: 11380).

The nucleic acids according to the invention can be prepared synthetically or obtained naturally or comprise a mixture of synthetic and natural nucleic acid constituents, and consist of various heterologous gene sections of various organisms.

As described above, synthetic nucleotide sequences with codons which are preferred from plants are preferred. These codons preferred from plants can be determined from codons having the highest protein frequency, which are expressed in the most interesting plant species.

In the preparation of an expression cassette, various DNA fragments can be manipulated in order to obtain a nucleotide sequence which expediently reads in the correct direction and and which is equipped with a correct reading frame. For the connection of the DNA fragments to one another, adapters or linkers can be added to the fragments.

Expediently, the promoter and the terminator regions can be provided in the transcription direction with a linker or polylinker which comprises one or more restriction sites for the insertion of this sequence. As a rule, the linker has 1 to 10, usually 1 to 8, preferably 2 to 6, restriction sites. In general, the linker within the regulatory areas has a size of less than 100 bp, frequently less than 60 bp, but at least 5 bp. The promoter can be either native or homologous, or foreign or heterologous for the host plant. The expression cassette preferably comprises, in the 5'-3' transcription direction, the promoter, a coding nucleic acid sequence or a nucleic acid construct and a region for transcriptional termination. Various termination regions are mutually arbitrarily exchangeable.

Examples of a terminator are the 35S terminator (Guerineau et al. (1988) Nucl Acids Res. 16: 11380), the nos terminator (Depicker A, Stachel S, Dhaese P, Zambryski P, Goodman HM. Nopaline synthase: transcript mapping and DNA sequence. J Mol Appl Genet. 1982;1(6):561-73) or the ocs terminator (Gielen, J, de Beuckeleer, M, Seurinck, J, Debroek, H, de Greve, H, Lemmers, M, van Montagu, M, Schell, J (1984) The complete sequence of the TL-DNA of the Agrobacterium tumefaciens plasmid pTiAch5. EMBO J. 3: 835-846).

Furthermore, manipulations which make available suitable restriction cleavage sites or remove the superfluous DNA or restriction cleavage sites can be employed. Where insertions, deletions or substitutions such as, for example, transitions and transversions are possible, in vitro mutagenesis, "primer repair", restriction or ligation can be used.

With suitable manipulations, such as, for example, restriction, "chewing-back" or filling of overhangs for "blunt ends", complementary ends of the fragments can be made available for ligation.

Preferred polyadenylation signals are plant polyadenylation signals, preferably those which essentially correspond to T-DNA polyadenylation signals from Agrobacterium tumefaciens, in particular of gene 3 of the T-DNA (octopine synthase) of the Ti plasmid pTiACH5 (Gielen et al., EMBO J. 3 (1984), 835 ff) or functional equivalents.

The transposition of foreign genes into the genome of a plant is described as transformation.

To this end, methods known per se for the transformation and regeneration of plants from plant tissues or plant cells can be utilized for transient or stable transformation.

Suitable methods for the transformation of plants are protoplast transformation by polyethylene glycol-induced DNA uptake, the biolistic process using the gene gun - the "particle bombardment" method, electroporation, the incubation of dry embryos in DNA-containing solution, microinjection and gene transfer mediated by Agrobacterium, described above. Said processes are described, for example, in B. Jenes et al., Techniques for Gene Transfer, in: Transgenic Plants, Vol. 1, Engineering and Utilization, edited by S.D. Kung and R. Wu, Academic Press (1993), 128-143, and in Potrykus, Annu. Rev. Plant Physiol. Plant Molec. Biol. 42 (1991), 205-225.

Preferably, the construct to be expressed is cloned in a vector which is suitable for transforming Agrobacterium tumefaciens, for example pBin19 (Bevan et al., Nucl. Acids Res. 12 (1984), 8711) or particularly preferably pSUN2, pSUN3, pSUN4 or pSUN5 (WO 02/00900).

Agrobacteria transformed using an expression plasmid can be used in a known manner for the transformation of plants, e.g. by bathing wounded leaves or leaf pieces or cotyledons in an Agrobacteria solution and subsequently culturing in suitable media.

For the preferred production of genetically modified plants, also called transgenic plants below, the fused expression cassette is cloned in a vector, for example pBin19 or in particular pSUN5 and pSUN3, which is suitable to be transformed into Agrobacterium tumefaciens. Agrobacteria transformed using such a vector can then be used in a known manner for the transformation of plants, in particular of crop plants, by, for example, bathing wounded leaves or pieces of leaf or cotyledons in an Agrobacteria solution and subsequently culturing in suitable media.

The transformation of plants by Agrobacteria is known, inter alia, from F.F. White, Vectors for Gene Transfer in Higher Plants; in Transgenic Plants, Vol. 1, Engineering and Utilization, edited by S.D. Kung and R. Wu, Academic Press, 1993, pp. 15-38. From the transformed cells of the wounded leaves or pieces of leaf or cotyledons, transgenic plants can be regenerated in a known manner which comprise one or more genes integrated into the expression cassette.

For the transformation of a host plant using one or more effect genes according to the invention, an expression cassette is incorporated into a recombinant vector as an insertion whose vector DNA comprises additional functional regulation signals, for example sequences for replication or integration. Suitable vectors are described, inter alia, in "Methods in Plant Molecular Biology and Biotechnology" (CRC Press), Chap. 6/7, pp. 71-119 (1993).

Using the recombination and cloning techniques cited above, the expression cassettes can be cloned into suitable vectors which make possible their proliferation, for example in E. coli. Suitable cloning vectors are, inter alia, pJIT117 (Guerineau et al. (1988) Nucl. Acids Res.16:11380), pBR332, pUC series, M13mp series and pACYC184. Particularly suitable are binary vectors, which can replicate both in E. coli and in Agrobacteria.

The invention therefore furthermore relates to a genetically modified plant of the genus Tagetes, comprising a promoter according to the invention and, functionally linked, to a gene to be expressed, with the proviso that genes from plants of the genus Tagetes, which are expressed in wild-type plants of the genus Tagetes by the respective promoter, are excluded.

Preferred promoters and preferred effect genes according to the invention are described above.

Particularly preferably, effect genes are selected from the group consisting of nucleic acids encoding a ketolase, nucleic acids encoding a beta-hydroxylase, nucleic acids encoding a beta-cyclase, nucleic acids encoding an epsilon-cyclase, nucleic acids encoding an epoxidase, nucleic acids encoding an HMG-CoA reductase, nucleic acids encoding an (E)-4-hydroxy-3-methylbut-2-enyl diphosphate reductase, nucleic acids encoding a 1-deoxy-D-xylose-5-phosphate synthase, nucleic acids encoding a 1-deoxy-D-xylose-5-phosphate reductoisomerase, nucleic acids encoding an isopentenyl diphosphate delta-isomerase, nucleic acids encoding a geranyl diphosphate synthase, nucleic acids encoding a farnesyl diphosphate synthase, nucleic acids encoding a geranylgeranyl diphosphate synthase, nucleic acids encoding a phytoene synthase, nucleic acids encoding a phytoene desaturase, nucleic acids encoding a prephytoene synthase, nucleic acids encoding a zeta-carotene desaturase, nucleic acids encoding a crtlSO protein, nucleic acids encoding an FtsZ protein and nucleic acids encoding a MinD protein.

Preferred, genetically modified plants of the genus Tagetes are Marigold, Tagetes erecta, Tagetes patula, Tagetes lucida, Tagetes pringlei, Tagetes palmeri, Tagetes minuta or Tagetes campanulata.

By means of the promoters according to the invention, it is possible with the aid of the processes according to the invention described above to regulate, in the genetically modified plants of the genus Tagetes according to the invention described above, the metabolic pathways to specific biosynthetic products.

To this end, for example, metabolic pathways which lead to a specific biosynthetic product are strengthened by causing or increasing the transcription rate or expression rate of genes of this biosynthesis pathway by the increased amount of protein leading to an increased total activity of these proteins of the desired biosynthesis pathway and thus by an increased metabolic flow to the desired biosynthetic product.

Depending on the desired biosynthetic product, the transcription rate or expression rate of various genes must be increased or reduced. As a rule, it is advantageous to modify the transcription rate or expression rate of several genes, i.e. to increase the transcription rate or expression rate of a combination of genes and/or to reduce the transcription rate or expression rate of a combination of genes.

In the genetically modified plants according to the invention, at least one increased or caused expression rate of a gene is to be attributed to a promoter according to the invention.

Further, additionally modified, i.e. additionally increased or additionally reduced, expression rate of further genes in genetically modified plants can, but do not have to, be attributed to the promoters according to the invention.

The invention therefore relates to a process for the preparation of biosynthetic products by culturing genetically modified plants of the genus Tagetes according to the invention.

The invention relates in particular to a process for the production of carotenoids by culturing genetically modified plants of the genus Tagetes according to the invention, wherein the genes to be expressed are selected from the group consisting of nucleic acids encoding a ketolase, nucleic acids encoding a beta-hydroxylase, nucleic acids encoding a beta-cyclase, nucleic acids encoding an epsilon-cyclase, nucleic acids encoding an epoxidase, nucleic acids encoding an HMG-CoA reductase, nucleic acids encoding an (E)-4-hydroxy-3-methylbut-2-enyl-diphosphate reductase, nucleic acids encoding a 1-deoxy-D-xylose-5-phosphate synthase, nucleic acids encoding a 1-deoxy-D-xylose-5-phosphate reductoisomerase, nucleic acids encoding an isopentenyl diphosphate delta-isomerase, nucleic acids encoding a geranyl diphosphate synthase, nucleic acids encoding a farnesyl diphosphate synthase, nucleic acids encoding a geranylgeranyl diphosphate synthase, nucleic acids encoding a phytoene synthase, nucleic acids encoding a phytoene desaturase, nucleic acids encoding a prephytoene synthase, nucleic acids encoding a zeta-carotene desaturase, nucleic acids encoding a crtISO protein, nucleic acids encoding an FtsZ protein and nucleic acids encoding a MinD protein.

The carotenoids are preferably selected from the group consisting of phytoene, phytofluene, lycopene, lutein, beta-carotin, zeaxanthin, astaxanthin, canthaxanthin, echinenone, 3-hydroxyechinenone, 3'-hydroxyechinenone, adonirubin, violaxanthin and adonixanthin.

In particular, the invention furthermore relates to a process for the production of ketocarotenoids by culturing genetically modified plants of the genus Tagetes according to the invention, wherein the genes to be expressed are selected from the group consisting of nucleic acids encoding a ketolase, nucleic acids encoding a beta-hydroxylase, nucleic acids encoding a beta-cyclase, nucleic acids encoding an epsilon-cyclase, nucleic acids encoding an epoxidase, nucleic acids encoding an HMG-CoA reductase, nucleic acids encoding an (E)-4-hydroxy-3-methylbut-2-enyl-diphosphate reductase, nucleic acids encoding a 1-deoxy-D-xylose-5-phosphate synthase, nucleic acids encoding a 1-deoxy-D-xylose-5-phosphate reductoisomerase, nucleic acids encoding an isopentenyl diphosphate delta-isomerase, nucleic acids encoding a geranyl diphosphate synthase, nucleic acids encoding a farnesyl diphosphate synthase, nucleic acids encoding a geranylgeranyl diphosphate synthase, nucleic acids encoding a phytoene synthase, nucleic acids encoding a phytoene desaturase, nucleic acids encoding a prephytoene synthase, nucleic acids encoding a zeta-carotene desaturase, nucleic acids encoding a crtISO protein, nucleic acids encoding an FtsZ protein and nucleic acids encoding a MinD protein.

The ketocarotenoids are preferably selected from the group consisting of astaxanthin, canthaxanthin, echinenone, 3-hydroxyechinenone, 3'-hydroxyechinenone, adonirubin, violaxanthin and adonixanthin.

In the process according to the invention for the preparation of biosynthetic products, in particular carotenoids, preferably ketocarotenoids, a harvesting of the plants and an isolation of the biosynthetic products, in particular carotenoids, preferably ketocarotenoids, from the plants, preferably from the petals of the plants, is preferably added to the culturing step of the genetically modified plants.

The genetically modified plants of the genus Tagetes are grown in a manner known per se on nutrient media and appropriately harvested.

The isolation of ketocarotenoids from the harvested flower leaves is carried out, for example in a manner known per se, for example by drying and subsequent extraction and, if appropriate, further chemical or physical purification processes, such as, for example, precipitation methods, crystallography, thermal separation processes, such as rectifying processes or physical separation processes, such as, for example, chromatography. The isolation of ketocarotenoids from the flower leaves is carried out, for example, preferably by means of organic solvents such as acetone, hexane, heptane, ether or tert-methyl butyl ether.

Further isolation processes of ketocarotenoids, in particular from flower leaves, are described, for example, in Egger and Kleinig (Phytochemistry (1967) 6, 437-440) and Egger (Phytochemistry (1965) 4, 609-618).

A particularly preferred ketocarotenoid is astaxanthin.

The ketocarotenoids are obtained in the process according to the invention in flower leaves in the form of their mono- or diesters with fatty acids. Some proven fatty acids are, for example, myristic acid, palmitic acid, stearic acid, oleic acid, linolenic acid, and lauric acid (Kamata and Simpson (1987) Comp. Biochem. Physiol. Vol. 86B(3), 587-591).

Genetically modified plants or plant parts according to the invention consumable by humans and animals, such as, in particular, flower leaves having an increased content of biosynthetic products, in particular carotenoids, in particular ketocarotenoids, in particular astaxanthin, can also be used, for example, directly or after processing known per se, as foodstuffs or feedstuffs or as feed and food supplements.

Furthermore, the genetically modified plants can be used for the production of biosynthetic product-, in particular carotenoid-, in particular ketocarotenoid-, in particular astaxanthin-containing extracts and/or for the production of feed and food supplements, and of cosmetics and pharmaceuticals.

The genetically modified plants of the genus Tagetes have, in comparison with the wild-type, an increased content of the desired biosynthetic products, in particular carotenoids, in particular ketocarotenoids, in particular astaxanthin.

An increased content is in this case also understood as meaning a caused content of ketocarotenoids, or astaxanthin.

Another embodiment of the invention is a process for producing a fish diet comprising drying petals from lutein-depleted Tagetes which express a ketolase encoding polynucleotide sequence as mentioned above. For this process the above mentioned dried petals from Tagetes can be mixed with known ingredients and auxiliaries for fish diets. The petal containing diet can be formulated in known manners in order to achieve a good mixture or bioavailability of the ingredients.

The invention is illustrated by the examples which now follow, but is not restricted to these:

### Experimental Section

### Example 1: Generation of lutein-depleted Tagetes plants

The generation of a lutein-depleted Tagetes plant has been disclosed in US 6,784,351 which is herewith completely incorporated by reference.

Naturally occurring Tagetes plants, which accumulate relatively high concentrations of beta-carotenoids while most of the alpha-carotenoids do not accumulate, are not known. Therefore, it was the task to develop a Tagetes plant which fulfills the requirements of i) being largely devoid of lutein and associated alpha-carotenoids in its flower petals and ii) being characterized by relatively high levels of total carotenoids.

The process how Tagetes plants with lutein-depleted flowers were created is described in the US patent No. 6,784,351. This patent describes in detail i) the EMS mutagenesis of Tagetes erecta "Scarletade" and "13819", and ii) the HPLC screening procedure to identify certain abnormal carotenoid profiles in flowers of "Scarletade" and "13819". The especially interesting mutant of Scarletade, 124-257, is described by its changed carotenoid profile in both petals and leaves.

### Example 2: Breeding of 31360-2-09-08 and 31360-2-09

*Tagetes erecta* selection 124-257, described in U.S. Patent No. 6,784,351, was found to have a low transformation rate using the identified tissue culture regeneration medium and Agrobacterium transformation technique. Using a standardized method, different plant selections can be transformed at different rates; therefore, to recover a target number of transformed plants, it can be expected that a selection having a low transformation rate would require use of a higher number of explants than a selection having a high transformation rate. A selection having a low transformation rate would require at least about 200 explants to recover about 1 transformed plant.

Instead of further optimizing the transformation protocol, a plant breeding backcross technique well known to those skilled in the art was used to transfer the mutation resulting in the increased zeaxanthin to lutein ratio of selection 124-257 to a selection having a higher transformation rate. Several *Tagetes erecta* marigold plants were identified as having acceptable transformation rates, and from these *Tagetes erecta* marigold plant named 13819 was selected. *Tagetes erecta* 13819 is a proprietary breeding selection of PanAmerican Seed located at 622 Town Road, West Chicago, Illinois 60185.

In the backcross program, selection 124-257 was used as the female parent in a cross with a selection of 13819 as the male parent. The resulting population was identified as 11754, and from this population a plant identified as 11754-2F was selected based on its hybrid characteristics. Plant 11754-2F was selfed and from this population plant identified as 11754-2F-1 was selected based on carotenoid profile and plant habit. Plant 11754-2F-1 was selfed and from this population plant identified as 11754-2F-1-2 was selected based on carotenoid profile and plant habit. Plant 11754-2F-1-2 was used as male parent in a cross with 13819 as the female parent. The resulting population was identified as 31360, and from this population a plant identified as 31360-2 was selected based on carotenoid profile and plant habit. Plant 31360-2 was selfed and from the resulting population, plants 31360-2-08 and 31360-2-09 were selected based on carotenoid profile, total carotenoid concentration, and plant habit. Both selections were selfed and seed from the cross was used to test transformation rates, and the seedlings from both selections were found to have acceptable transformation rates. In addition, the resulting plants from the selfed 31360-2-08 plant were found to be uniform for carotenoid profile, carotenoid concentration, and plant habit. The resulting plants from the selfed 31360-2-09 plant were found to segregate for total carotenoid concentration and plant habit characteristics. From the selfed 31360-2-09 population, a plant identified as 31360-2-09-08 was selected based on carotenoid profile, total carotenoid concentration, and plant habit. The selfed population from the 31360-2-09-08 plant was found to be uniform for carotenoid profile, carotenoid concentration, and to have an acceptable transformation rate.

### EXAMPLE 3: Synthesis of the coding sequence of the beta-carotene ketolase from Chlorella zofingiensis

The accession number BI934406 from the public sequence ("Chlorella zofingiensis beta-carotene ketolase/oxygenase (bkt/crto) gene, complete cds") was used to identify the coding sequence of of the beta-carotene ketolase from Chlorella zofingiensis. To isolate the DNA fragment described by SEQ ID NO: 1, the coding sequence of the beta-carotene ketolase from Chlorella (position 209-1147; = SEQ ID NO:2), fused to coding sequence of the transit peptide of the pisum sativum Rubisco smale subunit (rbcS) gene (position 38-205), was synthesized by the firm Entelechon GmbH (Regensburg, Germany).

### EXAMPLE 4: Cloning of the coding sequence of the beta-carotene ketolase from Scenedesmus vacuolatus Strain 211-8b from the culture collection of the Universtiy of Goettingen (SAG)

To isolate the DNA fragment described by **SEQ ID NO: 5,** *Scenedesmus vacuolatus SAG211-8b* was grown as for 14 days under low light conditions in basal media with peptone, as recommended by the culture collection of the University of Goettingen (SAG). RNA was isolated from tissue of *Scenedesmus vacuolatus* according to published methods (e.g. Maniatis T, Fritsch EF, and Sambrook J Molecular Cloning: A Laboratory Manual, Cold Spring Harbor Laboratory, Cold Spring Harbor (NY), (1989); Qiagen, *RNeasy Mini Handbook 06*/*2001*)*.* The isolated RNA was employed as matrix for cDNA synthesis according to published methods (e.g. Maniatis T, Fritsch EF, and Sambrook J Molecular Cloning: A Laboratory Manual, Cold Spring Harbor Laboratory, Cold Spring Harbor (NY), (1989)). The resulting cDNA was employed as matrix DNA for a polymerase chain reaction (PCR) mediated amplification using the oligonucleotide primers **SEQ ID NO: 3** and **SEQ ID NO: 4.** Alternatively, the fragment described by **SEQ ID NO: 5** can be generated by in vitro synthesis.
**SEQ ID NO 3:** SVK-10 gcgcatatggctcccaggcggcaa
**SEQ ID NO 4:** SVK-11 CGGTCGACTTACTCCACTACTGCTCC
**SEQ ID NO 5:** Scenesdesmus vacuolatus beta-carotene coding sequence
Position 1 to 24 Primer binding region for primer SVK-10
Position 985 to 1010 Primer binding region for primer SVK-11
Position 7 to 1002 region coding for beta-carotene ketoalse from Scenesdesmus vacuolatus (**=SEQ ID NO:6)**

PCR amplification was carried out as follows:
100 ng cDNA
1X PCR buffer
2,5 mM MgCl2,
200 µM each of dATP, dCTP, dGTP und dTTP
10 pmol of each oligonucleotide primers
2,5 Units Pfu DNA Polymerase (Stratagene)
in a final volume of 50 µl

The following temperature program is employed for the various amplifications (BIORAD Thermocycler).
1. 95°C for 5 min
2. 54°C for 1 min, followed by 72°C for 5 min and 95°C for 30 sec. Repeated 25 times.
3. 54°C for 1 min, followed by 72°C for 10min.
4. Storage at 4°C

The resulting PCR-products are cloned in a standard cloning vector and then reisolated by digestion with the suitable restriction endonucleases and cloned into a vector (predigested with the same enzymes) downstream of the promoter (e.g. CHRC promoter) and upstream of the terminator region (e.g. LB3 terminator) in operable linkage. This expression cassette is used to create vectors for stable transformation. This resulting vector is used for stable transformation of Tagetes, for example in germplasm described in example 1 and according to the procedure described in example 2. Biochemical analysis of petal tissue is performed as described in example 3.

### EXAMPLE 5 Vector Construction for Overexpression Experiments with the Ketolase from Chlorella zofingiensis and Scenedesmus vacuolatus SAG211-8b in Tagetes erecta

Vectors used for expression of the Scenedesmus vacuolatus & Chlorella zofingiensis beta-carotene ketolase genes in plants (overexpression) are designed to overexpress the ketolase under control of suitable promoters and are of two general types, biolistic and binary, depending on the plant transformation method to be used.

### For biolistic transformation (biolistic vectors), the requirements are as follows:

1. a backbone with a bacterial selectable marker (typically, an antibiotic resistance gene) and origin of replication functional in Escherichia coli (*E. coli ; e.g*., ColE1), and
2. a plant-specific portion consisting of:
   a. a gene expression cassette consisting of a promoter (e.g., ZmUBlint MOD), the gene of interest (typically, a full-length cDNA) and a transcriptional terminator (*e.g*., Agrobacterium tumefaciens nos terminator);
   b. a plant selectable marker cassette, consisting of a suitable promoter, selectable marker gene (*e.g*., pat; D-amino acid oxidase; dao1) and transcriptional terminator (eg. nos terminator).

Vectors designed for transformation by Agrobacterium tumefaciens (*A*. *tumefaciens;* binary vectors) consist of:
1. a backbone with a bacterial selectable marker functional in both E. coli and *A. tumefaciens* (*e.g.,* spectinomycin resistance mediated by the aadA gene) and two origins of replication, functional in each of aforementioned bacterial hosts, plus the *A. tumefaciens* virG gene;
2. a plant-specific portion as described for biolistic vectors above, except in this instance this portion is flanked by *A. tumefaciens* right and left border sequences which mediate transfer of the DNA flanked by these two sequences to the plant.

### Base Vector used for Cloning of Overexpression Constructs

**SEQ ID NO. 7: VC-LLL544-1qcz** (binary vector backbone)
Position 1 to 146 Right border
Position 320 to 1111 Adenyltransferase [aadA] gene coding region
Position 1560 to 2241 ColE1 *E. coli* origin of replication
Position 2615 to 2809 pVS1 origin (complementary)
Position 2413 to 5682 pVS1 replicon (complementary)
Position 5691 to 5905 Left border

### Vector Construction for Overexpression Experiments with Chlorella zofingiensis beta-carotene ketolase

**SEQ ID NO. 8:** T-DNA of binary vector with expression cassette BnPAPX:CZ BKT:CAT term (VC-SMS309)
Position 3 to 290 Nos (nopaline synthase) gene promoter
Position 303 to 854 Phosphinothricin Acetyltransferase synthetic gene/CDS Position 924 to 1176 Nos (nopaline synthase) gene terminator
Position 1367 to 1598 *Solanum tuberosum* Cathepsin D Inhibitor (CAT) terminator (complementary)
Position 1614 to 2552 *Chlorella zofingiensis* beta-carotene ketolase gene coding sequence (complementary)
Position 2556 to 2723 *Pisum sativum* Rubisco small subunit (RbcS) transit peptide coding sequence (complementary)
Position 2761 to 4798 *Arabidopsis thaliana* Triose phosphate translocator (TPT) promoter (complementary)

### Vector Construction for Overexpression Experiments with Scenedesmus vacuolatus beta-carotene ketolase

**SEQ ID NO. 9:** T-DNA of binary vector with expression cassette
BnPAPX:SV211 BKT:CAT term (VC-SMS330)
Position 3 to 290 Nos (nopaline synthase) gene promoter
Position 303 to 854 Phosphinothricin Acetyltransferase synthetic gene/CDS Position 924 to 1176 Nos (nopaline synthase) gene terminator
Position 1367 to 1598 *Solanum tuberosum* Cathepsin D Inhibitor (CAT) terminator (complementary)
Position 1660 to 2655 *Scenedesmus vacuolatus* SAG 211-8b beta-carotene ketolase gene coding sequence (complementary)
Position 2659 to 2826 *Pisum sativum* Rubisco small subunit (RbcS) transit peptide coding sequence (complementary) .
Position 2860 to 3890 *Brassica napus* Plastid-Associated protein X (PAPX) promoter (complementary)

### Example 8: Transformation of Tagetes 31360-2-08

Seeds of *Tagetes erecta* line 31360-2-08 were disinfected with 2% NaOCl solution for 10 minutes followed by three washes with autoclaved distilled water. Afterwards seeds were dried and can be stored under aseptic conditions at room temperature for a period of up to two weeks before *in vitro* germination. Germination occured on solidified MS medium (Murashige, T., and Skoog, F., A revised medium for rapid growth and bioassays with tobacco tissue cultures. Physiol. Plant. 15, 473-497,1962) in a 16/8 h light/darkness photoperiod for 1-3 weeks. Cotyledonary segments were prepared and used as primary target material for transformation. These segments were inoculated for 20 minutes in liquid MS medium containing *Agrobacterium tumefaciens* strain EHA105 [carrying the vector **VC-SMS330 or VC-SMS309]** cells at an OD₆₀₀ of 0.1. The construct **VC-SMS330 or VC-SMS309** contained the gene pat and allows therefore phosphinothricin (PPT) and/or BASTA selection. Explants are co-cultured for a period of 6 days on MS medium (pH 5.8) solidified with 0.8% agar and supplemented with 1 mg/l 3-indole-3-acetic acid (IAA), 3 mg/l indole-3-butyric acid (IBA), 500 mg/l 2-(N-morpholino) ethanesulfonic acid (MES) and 2% sucrose. Cultivation occured under controlled conditions at 21°C, 35-40 µmol m⁻² s⁻¹ white light intensity and 16/8 light/darkness rhythm. Shoots were induced on cotyledon explants on fresh MS medium, adjusted to pH 5.5, as described before supplemented with 500 mg/l Timentin, 1 mg/l PPT, 5 mg/l silver nitrate (AgNO₃). Second to fourth subcultures were done onto fresh MS medium following the formulation described above at pH 5.8 and 15 days subculture period. The newly formed shoot buds were transferred to a new medium to promote shoot regeneration. The shoot regeneration medium follows the formulation of MS supplemented with 0,7% agar, 250 mg/l Timentin, 1 mg/l PPT, 5 mg/l AgNO₃, 1 mg/l IAA, 3 mg/l 6-Benzylaminopurine (BAP), 500 mg/l MES and 2% sucrose and was adjusted to pH 5.8. Three subcultures were promoted in a 15 days subculture period. Regenerated shoots were then transferred onto elongation medium (MS) supplemented with 0.7% agar, 250 mg/l Timentin, 1 mg/l PPT, 5 mg/l AgNO₃, 0.5 mg/l IAA, 0.5 mg/l gibberellic acid (GA₃), 500 mg/l MES, 2% sucrose, pH 5.8. Three subcultures were performed, each for 15 days. Well elongated shoots (1.5 - 3.5 cm in length) with well expanded leaves were transferred onto rooting MS medium solidified with 0.7% agar and supplemented with 250 mg/l Timentin, 1 mg/l PPT, 0,5 mg/l IBA, 500 mg/l MES, 2% sucrose and adjusted to pH 5.8. Leaf material from rooted plants was analyzed by qPCR for the selection marker gene in order to confirm transgenicity and to determine the copy number of the construct integrated into the genome. After four weeks the well rooted transgenic shoots were transferred to ex vitro-conditions at the greenhouse. Hardening of plants in soil could be achieved with inverted funnels.They prevented dehydration of the plantlets. Afterwards plants were transferred into bigger pots with soil to promote growth and development until flowering under greenhouse conditions.

### Example 9:

### Enzymatic lipase-catalysed hydrolysis of carotenoid esters from plant extracts and identification of individual carotenoids

### a) General working procedure

Homogenized plant material from Tagetes, e.g. material of petals (30-100 mg fresh weight), was extracted with 100% acetone (three times with 500µl; each time with vigorous shaking for about 15 minutes). The combined solvent volumes were evaporated. Then, carotenoids were resuspended in 495 µl acetone and 4,95 ml potassium phosphate buffer (100 mM, pH 7.4) was added and intensely mixed. 17 mg Bile-salts (Sigma; the Bile-salts used are 1:1 mixtures of cholate and desoxycholate.) and 149 µl of a NaCl/CaCl₂-solution (3M NaCl and 75 mM CaCl₂) were added. The suspension was incubated for 30 minutes at 37°C. To hydrolyse the carotenoid esters enzymatically, 595 µl of a lipase solution (50 mg/ml lipase type 7 of *Candida rugosa* (Sigma) were added and incubated under constant shaking at 37°C. After 21 hours, again 595 µl lipase solution were added, followed by an incubation at 37°C for at least 5 hours. After the incubation, about 700 mg Na₂SO₄ (free of water) were added to the solution and solved. 1800 µl of petrolether was given to the solution and the carotenoids were extracted into the organic phase via vigorous and intensive mixing. The petrolether phase was taken off and fresh petrolether was added again, followed by vigorous mixing. This procedure was repeated till the organic phase stayed colorfree. The collected petrolether phases were combined and the petrolether was then evaporated in the vacuum. Released and therefore free carotenoids were solved in 100 -120 µl acetone. Using HPLC and a C30 reverse phase-column, carotenoids were identified based on their retention times and UV-VIS-spectra (in comparison to commercially available standard compounds).

### b) Method for low concentrations of carotenoid esters in plant material

Alternatively to the procedure described above, hydrolysis of carotenoid esters by lipase from *Candida rugosa* can be performed after separation of the extract on TLC plates (TLC = thin layer chromatography). 50-100mg fresh plant material was extracted three times with 750µl acetone. The combined organic supernatants were evaporated in the vacuum (elevated temperatures of 40-50°C can be tolerated). Extracted carotenoids were solved in 300µl petrolether:acetone (ratio 5:1) and well mixed. Solid particles were pelleted by centrifugation (15.000g) for 1-2 mintues. The upper phase was transferred to a new reaction tube. The remainder was re-extracted with 200µl petrolether:acetone (ratio 5:1) and particles were again removed by centrifugation. Supernatants were combined and the solvents were evaporated. The remainder was resuspended in 30µl petrolether:acetone (ratio 5:1) and spotted onto a TLC plate (thin layer chromatography plate; Silica-Gel 60, Merck, Germany). In case more than one spotting was required for preparative-analytical purposes, several aliquots each of 50-100 mg fresh weight should be prepared as described above.

The TLC plates were developed in petrolether:acetone (ratio 5:1). Bands of carotenoids could be identified visually due to their colors. Individual carotenoid bands were scraped out and sometimes pooled for preparative analysis. Carotenoids were dissolved from the Silica material with acetone; the solvent was later evaporated in the vacuum. The remainder was solved in 495µl acetone for the hydrolysis of carotenoid esters, 17mg Bile-salts (Sigma) and 4,95ml 0.1M potassium phosphate buffer (pH 7,4) and 149µl of (3M NaCl, 75mM CaCl₂) were added. After mixing all components well, the mixture was equilibrated for 30 minutes at 37°C. Then, 595µl lipase from *Candida rugosa* (Sigma, stock solution of 50mg/ml in 5mM CaCl₂) was added; incubation with lipase was done over night at 37°C under constant shaking. After 21 hours of incubation, an equal amount of lipase was added; the incubation proceeded for at least an additional 5 hours under constant shaking. Afterwards, 700mg Na₂SO₄ (free of water) were given to the solution and mixed well with 1800µl added petrolether for 1 minute; the mixture was centrifuged at 3500 g for 5 minutes. The upper phase was transferred into a new reaction vial, and the extraction of carotenoids into the petrolether phase was repeated several times. The extraction was finished when the upper phase remained colorless after mixing. The pooled petrolether phases were evaporated in the vacuum (temperatures of 40-50°C were acceptable). The remainder was solved in 120µl acetone, eventually with short sonication. The solved carotenoids were analyzed via HPLC using a C30-column and quantified using reference compounds as standards.

### KOH saponification of xanthophylls

10-20 mg fresh material of Tagetes petals were homogenized (via mortar and pestle in liquid nitrogen). The homogenous material was extracted with acetone, usually three times with 500ul acetone till the supernatant is colorless. If needed, material was shaken after each extraction. All supernatants were combined and evaporated to dryness using a speedvac concentrator. The pellet was dissolved in 180 µl of acetone and eventually briefly sonicated. For saponification, 20 µl of 10% KOH (in methanol) was added and incubated for 30 minutes under constant shaking (1000 rpm) in the dark at room temperature. The reaction was stopped by the addition of 20-30 µl 1M HCl (till neutral pH value was reached). Samples were centrifuged for 10 min at 13.000 rpm to pellet debris and analyzed by HPLC.

### Example 10: HPLC analysis of free carotenoids

The analysis of samples prepared according to the procedure described above was done under the following conditions:
HPLC conditions:
HPLC column: Prontosil C30, 250 x 4,6 mm, (Bischoff, Leonberg, Germany)
Flow rate: 1.0 ml/min
Eluents: Solvent A - 100% methanol
   Solvent B - 80% methanol, 0.2% ammoniumacetate
   Solvent C - 100% t-butyl-methylether
Detection: 300-530 nm

Gradienten profile:

| Time (min) | Flow rate | % Solvent A | % Solvent B | % Solvent C |
|---|---|---|---|---|
| 1.00 | 1.0 | 95.0 | 5.0 | 0 |
| 12.00 | 1.0 | 95.0 | 5.0 | 0 |
| 12.10 | 1.0 | 80.0 | 5.0 | 15.0 |
| 22.00 | 1.0 | 76.0 | 5.0 | 19.0 |
| 22.10 | 1.0 | 66.5 | 5.0 | 28.5 |
| 38.00 | 1.0 | 15.0 | 5.0 | 80.0 |
| 45.00 | 1.0 | 95.0 | 5.0 | 0 |
| 46.0 | 1.0 | 95.0 | 5.0 | 0 |

Some typical retention times for carotenoids are:
violaxanthin at about 11, 7 min, astaxanthin at about 17,7 min, adonixanthin at about 19 min, adonirubin at about 19,9 min, zeaxanthin at about 21 min.

After transformation of Tagetes explants of 31360-2-08 with the binary vector VC-SMS309, several transgenic plants were obtained which were named MS309-71, 197 & 137. After transformation of Tagetes explants of 31360-2-08 with the binary vector VC-SMS330, several transgenic plants were obtained which were named MS309-5, 15, 29 & 97. These plants were analyzed for individual yellow carotenoids, the natural occurring and endogenous xanthophylls, and the newly formed ketocarotenoids, especially for astaxanthin, canthaxanthin, echinenone, 3'-hydroxechinenone, 3-hydroxechinenone, phoenicoxanthin (=adonirubin) and adonixanthin.

**Table 1: Individual carotenoids in petals of transgenic Tagetes MS309**

| | ng / mg DW | | | % | | | |
|---|---|---|---|---|---|---|---|
| Plant | total caros | Ketos | Asta | A+P | A+P+C | bC+Cryp+Zea | Epoxides |
| MS309-71 | 13225 | 0,2 | 0,05 | 0,07 | 0,07 | 57,3 | 41,6 |
| MS309-107 | 12656 | 0,4 | 0,17 | 0,2 | 0,02 | 52,3 | 46,6 |
| MS309-137 | 12090 | 0,1 | 0,04 | 0,05 | 0,06 | 48,7 | 50,3 |

Listed values represent individual carotenoids in percent of total carotenoids, extracted and analyzed as described. The carotenoid extract was prepared from fully opened Tagets flowers. Value refer to fresh weight.

Legend for table 1 and following:
"total caros": total amounts of all carotenoids extracted from Tagets petals "Ketos": sum of all ketocarotenoids extracted from Tagetes petals (canthaxanthin, phoenicoxanthin, Aastaxanthin, adonixanthin, echinenone, 3'- und 3-hydroxyechinenone).
"Asta": designation for the ketocarotenoid astaxanthin
"Adoni": designation for the ketocarotenoid adonixanthin
"P" and ""Phoenico": designation for the ketocarotenoid phoenicoxanthin, also known as adonirubin
"C" and "Cantha": designation for the ketocarotenoid canthaxanthin "bC": designation for beta-carotene
"Cryp": designation fro beta-cryptoxanthin
"Zea": designation for zeaxanthin
"Cantha" designation for canthaxanthin
"3'-Hydroxy" and "HO-echi": designation for 3'-hydroxyechinenone "b-Crypto" bezeichnet beta-Cryptoxanthin
"Epoxides": designation for the combined concentration of the carotenoid epoxides violaxanthin, antheraxanthin and neoxanthin
"DW" stands for dry weight

**Table 2: Individual carotenoids in petals of transgenic Tagetes MS330**

| | ng/ mg DW | | | % | | | | | |
|---|---|---|---|---|---|---|---|---|---|
| Plant | total caros | Ketos | Asta | A+P | A+P+C | bC | Cryp | Zea | Epoxides |
| MS330-5 | 13410 | 20.7% | 16.7% | 18.8% | 20.5% | 67.9% | 0.3% | 1.2% | 1.0% |
| MS330-15 | 10459 | 20.3% | 14.6% | 17.6% | 20.0% | 66.9% | 0.2% | 1.4% | 1.6% |
| MS330-29 | 9961 | 35.4% | 27.3% | 32.1% | 35.0% | 54.6% | 0.2% | 1.6% | 1.6% |
| MS330-97 | 8525 | 22.8% | 17.0% | 20.1% | 22.4% | 67.4% | 0.2% | 1.5% | 1.4% |
| MS330-103 | 14813 | 21.2% | 15% | 18.5% | 21.0% | 71.6% | 0.1% | 1-1% | 0.6% |
| MS330-107 | 11395 | 20.1% | 14% | 17.4% | 19.8% | 68.5% | 0.1% | 1.1% | 0.8% |
| MS330-126 | 10856 | 23.2% | 16% | 20.1% | 22.8% | 65.7% | 0.2% | 0.9% | 1.2% |
| MS330-131 | 9064 | 19.5% | 15% | 17.5% | 19.4% | 69.9% | 0.2% | 1.1 % | 2.0% |
| MS330-137 | 11986 | 27.2% | 21% | 24.5% | 27.1% | 65.9% | 0.2% | 0.9% | 0.9% |
| SMS330-2 | 14012 | 20.8% | 16% | 18.6% | 20.6% | 64.8% | 0.2% | 0.9% | 2.6% |
| SMS330-20 | 13113 | 27.0% | 20% | 24.3% | 26.8% | 60.4% | 0.1% | 0.8% | 1.9% |
| SMS330-72 | 15119 | 21.0% | 16% | 18.4% | 20.7% | 69% | 0.2% | 1.0% | 1.6% |
| SMS330-92 | 16085 | 18.3% | 12% | 15.3% | 18.1% | 73.4% | 0.1% | 0.9% | 1.5% |
| SMS330-115 | 15460 | 21.1% | 16% | 18.3% | 20.8% | 68.7% | 0.1% | 1.0% | 1.0% |
| SMS330-133 | 8969 | 25.6% | 19% | 22.9% | 25.3% | 63.6% | 0.2% | 1.1% | 2.7% |
| SMS330-18 | 13604 | 16.0% | 9% | 13.1% | 15.9% | 59.5% | 0.3% | 4.3% | 14.5% |
| SMS330-68 | 12528 | 23.0% | 13% | 18.7% | 22.7% | 65.9% | 0.1% | 1.6% | 1.7% |
| SMS330-74 | 11707 | 20.3% | 16% | 18.5% | 20.1% | 65.3% | 0.2% | 0.9% | 3.3% |
| SMS330-87 | 19625 | 2.4% | 2% | 2.1% | 2.3% | 66.7% | 0.5% | 9% | 19.3% |
| SMS330-98 | 15402 | 23.6% | 17% | 20.8% | 23.3% | 68.1% | 0.1% | 0.7% | 1.1% |
| SMS330-112 | 13619 | 7.0% | 5% | 5.7% | 7.0% | 60% | 0.4% | 13% | 13.6% |

Listed values represent individual carotenoids in percent of total carotenoids, extracted and analyzed as described. The carotenoid extract was prepared from fully opened Tagetes flowers. Value refer to fresh weight.

**Table 3: Individual ketocarotenoids in petals of transgenic Tagetes MS330**

| Plant | Asta | Adoni | Phoenico | Cantha | HO-echi | Ketos |
|---|---|---|---|---|---|---|
| MS330-5 | 80.6% | 0.5% | 10.5% | 7.8% | 0.6% | 20.7% |
| MS330-15 | 71.7% | 0.6% | 14.6% | 12.0% | 1.1% | 20.3% |
| MS330-29 | 76.9% | 0.4% | 13.7% | 8.1% | 0.9% | 35.4% |
| MS330-97 | 74.8% | 0.3% | 13.7% | 10.2% | 1.1% | 22.8% |
| MS330-103 | 72.1% | 0.5% | 15.2% | 11.5% | 0.7% | 21.2% |
| MS330-107 | 69.6% | 0.5% | 16.6% | 12.1% | 1.1% | 20.1% |
| MS330-126 | 69.9% | 0.5% | 16.6% | 12.0% | 1.0% | 23.2% |
| MS330-131 | 78.5% | 0.4% | 11.0% | 9.6% | 0.5% | 19.5% |
| MS330-137 | 75.8% | 0.4% | 14.0% | 9.6% | 0.2% | 27.2% |
| SMS330-2 | 76.0% | 0.5% | 13.5% | 9.4% | 0.6% | 20.8% |
| SMS330-20 | 75.2% | 0.3% | 14.6% | 9.3% | 0.6% | 27.0% |
| SMS330-72 | 74.6% | 0.6% | 13.0% | 10.9% | 0.8% | 21.0% |
| SMS330-92 | 66.9% | 0.5% | 16.7% | 14.9% | 1.0% | 18.3% |
| SMS330-115 | 74.8% | 0.5% | 11.9% | 11.9% | 0.9% | 21.1% |
| SMS330-133 | 75.8% | 0.6% | 13.8% | 9.0% | 0.8% | 25.6% |
| SMS330-18 | 54.8% | 0.2% | 27.4% | 16.9% | 0.7% | 16.0% |
| SMS330-68 | 58.1% | 0.2% | 23.3% | 17.1% | 1.2% | 23.0% |
| SMS330-74 | 77.0% | 0.4% | 14.0% | 7.8% | 0.8% | 20.3% |
| SMS330-87 | 75.8% | 1.4% | 10.6% | 10.4% | 1.8% | 2.4% |
| SMS330-98 | 73.1% | 0.3% | 15.1% | 10.7% | 0.7% | 23.6% |
| SMS330-112 | 64.2% | 0.2% | 17.4% | 17.6% | 0.5% | 7.0% |

## Claims

1. A ketolase having a polypeptide sequence SEQ ID NO:6, a sequence derived from SEQ ID NO:6 by substitution, insertion or deletion of aminoacids, which has an identity of at least 60% with the respective sequence SEQ. ID NO:6.

2. A polynucleotide sequence encoding a ketolase according to claim 1.

3. A polynucleotide sequence according to claim 2 with the sequence SEQ ID NO:5.

4. A process for the production of keto-carotenoids in lutein-depleted plants of the genus Tagetes wherein a polynucleotide sequence according to claim 2 is used.

5. A process according to claim 4 wherein the polynucleotide sequence encoding a ketolase is under the transcriptional control of a plastid-lipid associated protein-promoter (PAP-promoter).

6. A process according to claim 5, wherein the PAP-promoter has the sequence SEQ ID NO:10 or SEQ ID NO:11.

7. A process according to claim 4, wherein expression takes place specifically in petals.

8. A process according to claim 4, wherein the keto-carotenoid is astaxanthin

9. A process according to claim 4, wherein a lutein-depleted Tagetes is cultured and the petals of the Tagetes are harvested.

10. A process for producing a fish diet comprising drying petals from lutein-depleted Tagetes which express a ketolase sequence according to claim 2.

## Patentansprüche

1. Ketolase mit einer Polypeptidsequenz SEQ ID NO:6, einer Sequenz, die von SEQ ID NO:6 durch Substitution, Insertion oder Deletion von Aminosäuren abgeleitet ist, die mindestens 60% Identität zu der entsprechenden Sequenz SEQ ID NO:6 aufweist.

2. Polynukleotidsequenz, die für eine Ketolase nach Anspruch 1 codiert.

3. Polynukleotidsequenz nach Anspruch 2 mit der Sequenz SEQ ID NO:5.

4. Verfahren zur Herstellung von Keto-Karotinoiden in luteinabgereicherten Pflanzen der Gattung Tagetes, bei dem eine Polynukleotidsequenz nach Anspruch 2 verwendet wird.

5. Verfahren nach Anspruch 4, wobei die Polynukleotidsequenz, die für eine Ketolase codiert, unter der transkriptionellen Kontrolle eines PAP-Promotors (Plastid Lipid Associated Protein Promoter) steht.

6. Verfahren nach Anspruch 5, wobei der PAP-Promotor die Sequenz SEQ ID NO:10 oder SEQ ID NO:11 aufweist.

7. Verfahren nach Anspruch 4, wobei die Expression spezifisch in Blütenblättern stattfindet.

8. Verfahren nach Anspruch 4, wobei es sich bei dem Keto-Karotinoid um Astaxanthin handelt.

9. Verfahren nach Anspruch 4, wobei eine luteinabgereicherte Tagetes kultiviert wird und die Blütenblätter der Tagetes geerntet werden.

10. Verfahren zur Herstellung eines Fischfutters, das das Trocknen von Blütenblättern von luteinabgereicherten Tagetes, die eine Ketolasesequenz nach Anspruch 2 exprimieren, umfasst.

## Revendications

1. Cétolase ayant une séquence polypeptidique SEQ ID n° : 6, une séquence dérivée de la SEQ ID n° : 6 par substitution, insertion ou délétion d'acides aminés qui a une identité d'au moins 60% avec la séquence SEQ ID n° : 6 respective.

2. Séquence de polynucléotides codant pour une cétolase selon la revendication 1.

3. Séquence de polynucléotides, selon la revendication 2, avec la séquence SEQ ID n° : 5.

4. Processus pour la production de céto-caroténoïdes dans des plantes dépourvues de lutéine du genre Tagètes, dans lequel on utilise la séquence de polynucléotides selon la revendication 2.

5. Processus selon la revendication 4, dans lequel la séquence de polynucléotides codant pour une cétolase est sous le contrôle transcriptionnel d'un promoteur de protéine associé à un lipide de plaste (promoteur PAP).

6. Processus selon la revendication 5, dans lequel le promoteur PAP a la séquence SEQ ID n° : 10 ou SEQ ID n° : 11.

7. Processus selon la revendication 4, dans lequel l'expression a lieu spécifiquement dans les pétales.

8. Processus selon la revendication 4, dans lequel le céto-caroténoïde est l'astaxanthine.

9. Processus selon la revendication 4, dans lequel une Tagète dépourvue de lutéine est cultivée et les pétales de la Tagète sont cueillies.

10. Processus de production d'un régime à base de poisson comprenant l'étape consistant à sécher des pétales provenant d'une Tagète dépourvue de lutéine, qui exprime une séquence de cétolase selon la revendication 2.


### SEQUENCE LISTING

<110> BASF Plant Science GmbH
<120> New ketolases for the production of ketocarotenoids in Tagetes
<130> PF 0000058583
<160> 11
<170> PatentIn version 3.1
<210> 1
   <211> 1153
   <212> DNA
   <213> Chlorella zofingiensis
<220>
<221> CDS
<222> (209)..(1147)
   <223> beta carotene ketolase
<400> 1
   ggatccaagc tttgcaattc atacagaagt gagaaaaatg gcttctatga tatcctcttc      60
   agctgtgact acagtcagcc gtgcttctac ggtgcaatcg gccgcggtgg ctccattcgg     120
   cggcctcaaa tccatgactg gattcccagt taagaaggtc aacactgaca ttacttccat     180
   tacaagcaat ggtggaagag taaagtgc atg gcg cca gat gtg aca cat gtg        232
                                  Met Ala Pro Asp Val Thr His Val
                                  1               5
   cag cca cgt gta cag tcc ccg gct ggc ccc gat gat gag gat gac gcg       280
   Gln Pro Arg Val Gln Ser Pro Ala Gly Pro Asp Asp Glu Asp Asp Ala
       10                  15                  20
   tta agc ttg tgg aaa gcc caa tat cct atg ccg gag gag aag ggt aca       328
   Leu Ser Leu Trp Lys Ala Gln Tyr Pro Met Pro Glu Glu Lys Gly Thr
   25                  30                  35                  40
   gta tcc aaa cct caa gcc gca ctc aaa tac agg cca cca cgc agt gac       376
   Val Ser Lys Pro Gln Ala Ala Leu Lys Tyr Arg Pro Pro Arg Ser Asp
                   45                  50                  55
   tgg aag ggt gta tca att gca tgc act gtc atc acc cta tgg aca gct       424
   Trp Lys Gly Val Ser Ile Ala Cys Thr Val Ile Thr Leu Trp Thr Ala
               60                  65                  70
   gtc ttt tac cat ggc tgc tgg caa atc aaa ctc aca ggc cct gat aag       472
   Val Phe Tyr His Gly Cys Trp Gln Ile Lys Leu Thr Gly Pro Asp Lys
           75                  80                  85
   tca gcc tgg tgg gac gtt gtt gca acg ttt ctg gca ctg gag ttc ctc       520
   Ser Ala Trp Trp Asp Val Val Ala Thr Phe Leu Ala Leu Glu Phe Leu
       90                  95                  100
   aac act ggg ctt ttc atc acc acg cat gat gcc atg cat ggg act att       568
   Asn Thr Gly Leu Phe Ile Thr Thr His Asp Ala Met His Gly Thr Ile
   105                 110                 115                 120
   gcc atc agg aac cgt cgt ttg aat gac cta ctt ggc aat ata gcc atc      616
   Ala Ile Arg Asn Arg Arg Leu Asn Asp Leu Leu Gly Asn Ile Ala Ile
                   125                 130                 135
   agc cta tat gcc tgg ttt gac tat gac atg ctg cac aag aag cac tgg      664
   Ser Leu Tyr Ala Trp Phe Asp Tyr Asp Met Leu His Lys Lys His Trp
               140                 145                 150
   gag cat cac aac ttc act ggg tta cca cat aaa gac cca gac ttc cat      712
   Glu His His Asn Phe Thr Gly Leu Pro His Lys Asp Pro Asp Phe His
           155                 160                 165
   cga ggc gat cct gcg cta cat aag tgg ttt ggc agg ttt atg tgg gag      760
   Arg Gly Asp Pro Ala Leu His Lys Trp Phe Gly Arg Phe Met Trp Glu
       170                 175                 180
   tat gca aca cca ctc cag ttt gcc aag atc ttc gca tac ccc ttc ttc      808
   Tyr Ala Thr Pro Leu Gln Phe Ala Lys Ile Phe Ala Tyr Pro Phe Phe
   185                 190                 195                 200
   cta caa tcc tta cgg gtg caa tac ccc aat tta tgc gtc ttt ctg gcg      856
   Leu Gln Ser Leu Arg Val Gln Tyr Pro Asn Leu Cys Val Phe Leu Ala
                   205                 210                 215
   gct gca ccc ctg gtc agt gcg ttc cga ttg ttc tat ttt ggc acc tat      904
   Ala Ala Pro Leu Val Ser Ala Phe Arg Leu Phe Tyr Phe Gly Thr Tyr
               220                 225                 230
   tta ccc cac ctc ccc tcc aat gct cag gag aca atg ccc tgg gag aaa      952
   Leu Pro His Leu Pro Ser Asn Ala Gln Glu Thr Met Pro Trp Glu Lys
           235                 240                 245
   tct cac agt gct gat gac cct cgg ccg ctg tca ttc ttg aaa tgt tat     1000
   Ser His Ser Ala Asp Asp Pro Arg Pro Leu Ser Phe Leu Lys Cys Tyr
       250                 255                 260
   cac ttt gat tat cac tgg gag cat cac agg tgg cct tat gcc cct tgg     1048
   His Phe Asp Tyr His Trp Glu His His Arg Trp Pro Tyr Ala Pro Trp
   265                 270                 275                 280
   tgg gag tta ccc gtg tgt aag cgc atc aca aag aca ctg gat gct gca     1096
   Trp Glu Leu Pro Val Cys Lys Arg Ile Thr Lys Thr Leu Asp Ala Ala
                   285                 290                 295
   gtt cca gga gta cag tca gac ggc acg aag aag agt cag ttg gtg aac     1144
   Val Pro Gly Val Gln Ser Asp Gly Thr Lys Lys Ser Gln Leu Val Asn
               300                 305                 310
   taa gaattc                                                          1153
<210> 2
   <211> 312
   <212> PRT
   <213> Chlorella zofingiensis
<400> 2
   Met Ala Pro Asp Val Thr His Val Gln Pro Arg Val Gln Ser Pro Ala
   1               5                   10                  15
   Gly Pro Asp Asp Glu Asp Asp Ala Leu Ser Leu Trp Lys Ala Gln Tyr
               20                  25                  30
   Pro Met Pro Glu Glu Lys Gly Thr Val Ser Lys Pro Gln Ala Ala Leu
           35                  40                  45
   Lys Tyr Arg Pro Pro Arg Ser Asp Trp Lys Gly Val Ser Ile Ala Cys
       50                  55                  60
   Thr Val Ile Thr Leu Trp Thr Ala Val Phe Tyr His Gly Cys Trp Gln
   65                  70                  75                  80
   Ile Lys Leu Thr Gly Pro Asp Lys Ser Ala Trp Trp Asp Val Val Ala
                   85                  90                  95
   Thr Phe Leu Ala Leu Glu Phe Leu Asn Thr Gly Leu Phe Ile Thr Thr
               100                 105                 110
   His Asp Ala Met His Gly Thr Ile Ala Ile Arg Asn Arg Arg Leu Asn
           115                 120                 125
   Asp Leu Leu Gly Asn Ile Ala Ile Ser Leu Tyr Ala Trp Phe Asp Tyr
       130                 135                 140
   Asp Met Leu His Lys Lys His Trp Glu His His Asn Phe Thr Gly Leu
   145                 150                 155                 160
   Pro His Lys Asp Pro Asp Phe His Arg Gly Asp Pro Ala Leu His Lys
                   165                 170                 175
   Trp Phe Gly Arg Phe Met Trp Glu Tyr Ala Thr Pro Leu Gln Phe Ala
               180                 185                 190
   Lys Ile Phe Ala Tyr Pro Phe Phe Leu Gln Ser Leu Arg Val Gln Tyr
           195                 200                 205
   Pro Asn Leu Cys Val Phe Leu Ala Ala Ala Pro Leu Val Ser Ala Phe
       210                 215                 220
   Arg Leu Phe Tyr Phe Gly Thr Tyr Leu Pro His Leu Pro Ser Asn Ala
   225                 230                 235                 240
   Gln Glu Thr Met Pro Trp Glu Lys Ser His Ser Ala Asp Asp Pro Arg
                   245                 250                 255
   Pro Leu Ser Phe Leu Lys Cys Tyr His Phe Asp Tyr His Trp Glu His
               260                 265                 270
   His Arg Trp Pro Tyr Ala Pro Trp Trp Glu Leu Pro Val Cys Lys Arg
           275                 280                 285
   Ile Thr Lys Thr Leu Asp Ala Ala Val Pro Gly Val Gln Ser Asp Gly
       290                 295                 300
   Thr Lys Lys Ser Gln Leu Val Asn
   305                 310
<210> 3
   <211> 24
   <212> DNA
   <213> Artificial
<400> 3
   gcgcatatgg ctcccaggcg gcaa 24
<210> 4
   <211> 26
   <212> DNA
   <213> Artificial
<400> 4
   cggtcgactt actccactac tgctcc 26
<210> 5
   <211> 1010
   <212> DNA
   <213> Scenedesmus vacuolatus
<220>
   <221> CDS
   <222> (7)..(1002)
   <223>
<400> 5
   gcgcat atg gct ccc agg cgg caa tca acg ctg ccg cag cag acc aaa        48
          Met Ala Pro Arg Arg Gln Ser Thr Leu Pro Gln Gln Thr Lys
          1               5                   10
   gct ggc tct cca acc agt ggc tca gat gct gcc atc cct gag ccc gat       96
   Ala Gly Ser Pro Thr Ser Gly Ser Asp Ala Ala Ile Pro Glu Pro Asp
   15                  20                  25                  30
   gtc atc gac gtg tgg aaa gcg caa tac cct ctg ccg gat gaa aat gta      144
   Val Ile Asp Val Trp Lys Ala Gln Tyr Pro Leu Pro Asp Glu Asn Val
                   35                  40                  45
   gca ggg agc atg aat gag gtc aag cag ttg tac agg cca cct cgc aat      192
   Ala Gly Ser Met Asn Glu Val Lys Gln Leu Tyr Arg Pro Pro Arg Asn
                   50                  55                  60
   gat gtg aag ggc ata agc att gcc ttg ggc ctg att gca gcc tgg tgc      240
   Asp Val Lys Gly Ile Ser Ile Ala Leu Gly Leu Ile Ala Ala Trp Cys
               65                  70                  75
   gtg ctg ttt tac cac ggc tgc tgg cag atc cag ctg tct ggc agt cag      288
   Val Leu Phe Tyr His Gly Cys Trp Gln Ile Gln Leu Ser Gly Ser Gln
           80                  85                  90
   cgc tcc tgg tgg att gac att gct ggc aca ttt att ttg ttg gag ttc      336
   Arg Ser Trp Trp Ile Asp Ile Ala Gly Thr Phe Ile Leu Leu Glu Phe
   95                  100                 105                 110
   gtc aac aca ggc ctt ttc atc acc acg cac gat gcc atg cat ggc act      384
   Val Asn Thr Gly Leu Phe Ile Thr Thr His Asp Ala Met His Gly Thr
                   115                 120                 125
   gtt tgt tac agg aac cgc aag ctg aac gat ctg ttg ggt cgt ata gcc      432
   Val Cys Tyr Arg Asn Arg Lys Leu Asn Asp Leu Leu Gly Arg Ile Ala
               130                 135                 140
   atc aca ctg tac gcc tgg ttt gac tac gac atg ctt cac agg aag cac      480
   Ile Thr Leu Tyr Ala Trp Phe Asp Tyr Asp Met Leu His Arg Lys His
           145                 150                 155
   tgg gag cat cac aac tac aca gga cag aag ggc aaa gac cct gac ttc      528
   Trp Glu His His Asn Tyr Thr Gly Gln Lys Gly Lys Asp Pro Asp Phe
       160                 165                 170
   cac agg ggc aac cct gca ctg cca gtg tgg tat gcc agg ttc atg tgg      576
   His Arg Gly Asn Pro Ala Leu Pro Val Trp Tyr Ala Arg Phe Met Trp
   175                 180                 185                 190
   gaa tac tcc acc ccc ttg cag ttt gcc aaa atc atc ctg gtg agt cag      624
   Glu Tyr Ser Thr Pro Leu Gln Phe Ala Lys Ile Ile Leu Val Ser Gln
                   195                 200                 205
   gtg ctg caa gcc ctg gga gtg ccc tac aac aac ctg tgt gtc tac atg      672
   Val Leu Gln Ala Leu Gly Val Pro Tyr Asn Asn Leu Cys Val Tyr Met
               210                 215                 220
   gct gct gcg ccc ctg gtg gcc gcc ttc agg ctg ttc tat ttt ggc acc      720
   Ala Ala Ala Pro Leu Val Ala Ala Phe Arg Leu Phe Tyr Phe Gly Thr
           225                 230                 235
   tac ctg ccg cac ttg ccc ccc aac gcc cag gag gtg atg gtg tgg cag      768
   Tyr Leu Pro His Leu Pro Pro Asn Ala Gln Glu Val Met Val Trp Gln
       240                 245                 250
   aag agt cac tct agt gat gct ccc tcc tgg ctg tct ttc ctc aag tgt      816
   Lys Ser His Ser Ser Asp Ala Pro Ser Trp Leu Ser Phe Leu Lys Cys
   255                 260                 265                 270
   tac cac ttt gat tat cat tgg gaa cac cac aga tgg cca tat gct ccc      864
   Tyr His Phe Asp Tyr His Trp Glu His His Arg Trp Pro Tyr Ala Pro
                   275                 280                 285
   tgg tgg gag ttg ccg aag gcg aag aaa att aca caa caa act cag cat      912
   Trp Trp Glu Leu Pro Lys Ala Lys Lys Ile Thr Gln Gln Thr Gln His
               290                 295                 300
   cac caa caa acc aag cag cag cag ccc atg cag cag gca aaa gcg cag      960
   His Gln Gln Thr Lys Gln Gln Gln Pro Met Gln Gln Ala Lys Ala Gln
           305                 310                 315
   gtt gtc tcc cag ctg gcc cct gca gga gca gta gtg gag taa gtcgaccg    1010
   Val Val Ser Gln Leu Ala Pro Ala Gly Ala Val Val Glu
       320                 325                 330
<210> 6
   <211> 331
   <212> PRT
   <213> Scenedesmus vacuolatus
<400> 6
   Met Ala Pro Arg Arg Gln Ser Thr Leu Pro Gln Gln Thr Lys Ala Gly
   1               5                   10                  15
   Ser Pro Thr Ser Gly Ser Asp Ala Ala Ile Pro Glu Pro Asp Val Ile
               20                  25                  30
   Asp Val Trp Lys Ala Gln Tyr Pro Leu Pro Asp Glu Asn Val Ala Gly
           35                  40                  45
   Ser Met Asn Glu Val Lys Gln Leu Tyr Arg Pro Pro Arg Asn Asp Val
       50                  55                  60
   Lys Gly Ile Ser Ile Ala Leu Gly Leu Ile Ala Ala Trp Cys Val Leu
   65                  70                  75                  80
   Phe Tyr His Gly Cys Trp Gln Ile Gln Leu Ser Gly Ser Gln Arg Ser
                   85                  90                  95
   Trp Trp Ile Asp Ile Ala Gly Thr Phe Ile Leu Leu Glu Phe Val Asn
               100                 105                 110
   Thr Gly Leu Phe Ile Thr Thr His Asp Ala Met His Gly Thr Val Cys
           115                 120                 125
   Tyr Arg Asn Arg Lys Leu Asn Asp Leu Leu Gly Arg Ile Ala Ile Thr
       130                 135                 140
   Leu Tyr Ala Trp Phe Asp Tyr Asp Met Leu His Arg Lys His Trp Glu
   145                 150                 155                 160
   His His Asn Tyr Thr Gly Gln Lys Gly Lys Asp Pro Asp Phe His Arg
                   165                 170                 175
   Gly Asn Pro Ala Leu Pro Val Trp Tyr Ala Arg Phe Met Trp Glu Tyr
               180                 185                 190
   Ser Thr Pro Leu Gln Phe Ala Lys Ile Ile Leu Val Ser Gln Val Leu
           195                 200                 205
   Gln Ala Leu Gly Val Pro Tyr Asn Asn Leu Cys Val Tyr Met Ala Ala
       210                 215                 220
   Ala Pro Leu Val Ala Ala Phe Arg Leu Phe Tyr Phe Gly Thr Tyr Leu
   225                 230                 235                 240
   Pro His Leu Pro Pro Asn Ala Gln Glu Val Met Val Trp Gln Lys Ser
                   245                 250                 255
   His Ser Ser Asp Ala Pro Ser Trp Leu Ser Phe Leu Lys Cys Tyr His
               260                 265                 270
   Phe Asp Tyr His Trp Glu His His Arg Trp Pro Tyr Ala Pro Trp Trp
           275                 280                 285
   Glu Leu Pro Lys Ala Lys Lys Ile Thr Gln Gln Thr Gln His His Gln
       290                 295                 300
   Gln Thr Lys Gln Gln Gln Pro Met Gln Gln Ala Lys Ala Gln Val Val
   305                 310                 315                 320
   Ser Gln Leu Ala Pro Ala Gly Ala Val Val Glu
                   325                 330
<210> 7
   <211> 5905 <212> DNA
   <213> Artificial
<400> 7
   gattgtcgtt tcccgccttc agtttaaact atcagtgttt gacaggatat attggcgggt     60
   aaacctaaga gaaaagagcg tttattagaa taatcggata tttaaaaggg cgtgaaaagg    120
   tttatccgtt cgtccatttg tatgtccatg gaacgcagtg gcggttttca tggcttgtta    180
   tgactgtttt tttggggtac agtctatgcc tcgggcatcc aagcagcaag cgcgttacgc    240
   cgtgggtcga tgtttgatgt tatggagcag caacgatgtt acgcagcagg gcagtcgccc    300
   taaaacaaag ttaaacatca tgggggaagc ggtgatcgcc gaagtatcga ctcaactatc    360
   agaggtagtt ggcgtcatcg agcgccatct cgaaccgacg ttgctggccg tacatttgta    420
   cggctccgca gtggatggcg gcctgaagcc acacagtgat attgatttgc tggttacggt    480
   gaccgtaagg cttgatgaaa caacgcggcg agctttgatc aacgaccttt tggaaacttc 540
   ggcttcccct ggagagagcg agattctccg cgctgtagaa gtcaccattg ttgtgcacga 600
   cgacatcatt ccgtggcgtt atccagctaa gcgcgaactg caatttggag aatggcagcg 660
   caatgacatt cttgcaggta tcttcgagcc agccacgatc gacattgatc tggctatctt 720
   gctgacaaaa gcaagagaac atagcgttgc cttggtaggt ccagcggcgg aggaactctt 780
   tgatccggtt cctgaacagg atctatttga ggcgctaaat gaaaccttaa cgctatggaa 840
   ctcgccgccc gactgggctg gcgatgagcg aaatgtagtg cttacgttgt cccgcatttg 900
   gtacagcgca gtaaccggca aaatcgcgcc gaaggatgtc gctgccgact gggcaatgga 960
   gcgcctgccg gcccagtatc agcccgtcat acttgaagct agacaggctt atcttggaca 1020
   agaagaagat cgcttggcct cgcgcgcaga tcagttggaa gaatttgtcc actacgtgaa 1080
   aggcgagatc accaaggtag tcggcaaata atgtctagct agaaattcgt tcaagccgac 1140
   gccgcttcgc ggcgcggctt aactcaagcg ttagatgcac taagcacata attgctcaca 1200
   gccaaactat caggtcaagt ctgcttttat tatttttaag cgtgcataat aagccctaca 1260
   caaattggga gatatatcat gcatgaccaa aatcccttaa cgtgagtttt cgttccactg 1320
   agcgtcagac cccgtagaaa agatcaaagg atcttcttga gatccttttt ttctgcgcgt 1380
   aatctgctgc ttgcaaacaa aaaaaccacc gctaccagcg gtggtttgtt tgccggatca 1440
   agagctacca actctttttc cgaaggtaac tggcttcagc agagcgcaga taccaaatac 1500
   tgtccttcta gtgtagccgt agttaggcca ccacttcaag aactctgtag caccgcctac 1560
   atacctcgct ctgctaatcc tgttaccagt ggctgctgcc agtggcgata agtcgtgtct 1620
   taccgggttg gactcaagac gatagttacc ggataaggcg cagcggtcgg gctgaacggg 1680
   gggttcgtgc acacagccca gcttggagcg aacgacctac accgaactga gatacctaca 1740
   gcgtgagcta tgagaaagcg ccacgcttcc cgaagggaga aaggcggaca ggtatccggt 1800
   aagcggcagg gtcggaacag gagagcgcac gagggagctt ccagggggaa acgcctggta 1860
   tctttatagt cctgtcgggt ttcgccacct ctgacttgag cgtcgatttt tgtgatgctc 1920
   gtcagggggg cggagcctat ggaaaaacgc cagcaacgcg gcctttttac ggttcctggc 1980
   cttttgctgg ccttttgctc acatgttctt tcctgcgtta tcccctgatt ctgtggataa 2040
   ccgtattacc gcctttgagt gagctgatac cgctcgccgc agccgaacga ccgagcgcag 2100
   cgagtcagtg agcgaggaag cggaagagcg cctgatgcgg tattttctcc ttacgcatct 2160
   gtgcggtatt tcacaccgca tatggtgcac tctcagtaca atctgctctg atgccgcata 2220
   gttaagccag tatacactcc gctatcgcta cgtgactggg tcatggctgc gccccgacac 2280
   ccgccaacac ccgctgacgc gccctgacgg gcttgtctgc tcccggcatc cgcttacaga 2340
   caagctgtga ccgtctccgg gagctgcatg tgtcagaggt tttcaccgtc atcaccgaaa 2400
   cgcgcgaggc agggtgcctt gatgtgggcg ccggcggtcg agtggcgacg gcgcggcttg 2460
   tccgcgccct ggtagattgc ctggccgtag gccagccatt tttgagcggc cagcggccgc 2520
   gataggccga cgcgaagcgg cggggcgtag ggagcgcagc gaccgaaggg taggcgcttt 2580
   ttgcagctct tcggctgtgc gctggccaga cagttatgca caggccaggc gggttttaag 2640
   agttttaata agttttaaag agttttaggc ggaaaaatcg ccttttttct cttttatatc 2700
   agtcacttac atgtgtgacc ggttcccaat gtacggcttt gggttcccaa tgtacgggtt 2760
   ccggttccca atgtacggct ttgggttccc aatgtacgtg ctatccacag gaaagagacc 2820
   ttttcgacct ttttcccctg ctagggcaat ttgccctagc atctgctccg tacattagga 2880
   accggcggat gcttcgccct cgatcaggtt gcggtagcgc atgactagga tcgggccagc 2940
   ctgccccgcc tcctccttca aatcgtactc cggcaggtca tttgacccga tcagcttgcg 3000
   cacggtgaaa cagaacttct tgaactctcc ggcgctgcca ctgcgttcgt agatcgtctt 3060
   gaacaaccat ctggcttctg ccttgcctgc ggcgcggcgt gccaggcggt agagaaaacg 3120
   gccgatgccg ggatcgatca aaaagtaatc ggggtgaacc gtcagcacgt ccgggttctt 3180
   gccttctgtg atctcgcggt acatccaatc agctagctcg atctcgatgt actccggccg 3240
   cccggtttcg ctctttacga tcttgtagcg gctaatcaag gcttcaccct cggataccgt 3300
   caccaggcgg ccgttcttgg ccttcttcgt acgctgcatg gcaacgtgcg tggtgtttaa 3360
   ccgaatgcag gtttctacca ggtcgtcttt ctgctttccg ccatcggctc gccggcagaa 3420
   cttgagtacg tccgcaacgt gtggacggaa cacgcggccg ggcttgtctc ccttcccttc 3480
   ccggtatcgg ttcatggatt cggttagatg ggaaaccgcc atcagtacca ggtcgtaatc 3540
   ccacacactg gccatgccgg ccggccctgc ggaaacctct acgtgcccgt ctggaagctc 3600
   gtagcggatc acctcgccag ctcgtcggtc acgcttcgac agacggaaaa cggccacgtc 3660
   catgatgctg cgactatcgc gggtgcccac gtcatagagc atcggaacga aaaaatctgg 3720
   ttgctcgtcg cccttgggcg gcttcctaat cgacggcgca ccggctgccg gcggttgccg 3780
   ggattctttg cggattcgat cagcggccgc ttgccacgat tcaccggggc gtgcttctgc 3840
   ctcgatgcgt tgccgctggg cggcctgcgc ggccttcaac ttctccacca ggtcatcacc 3900
   cagcgccgcg ccgatttgta ccgggccgga tggtttgcga ccgctcacgc cgattcctcg 3960
   ggcttggggg ttccagtgcc attgcagggc cggcagacaa cccagccgct tacgcctggc 4020
   caaccgcccg ttcctccaca catggggcat tccacggcgt cggtgcctgg ttgttcttga 4080
   ttttccatgc cgcctccttt agccgctaaa attcatctac tcatttattc atttgctcat 4140
   ttactctggt agctgcgcga tgtattcaga tagcagctcg gtaatggtct tgccttggcg 4200
   taccgcgtac atcttcagct tggtgtgatc ctccgccggc aactgaaagt tgacccgctt 4260
   catggctggc gtgtctgcca ggctggccaa cgttgcagcc ttgctgctgc gtgcgctcgg 4320
   acggccggca cttagcgtgt ttgtgctttt gctcattttc tctttacctc attaactcaa 4380
   atgagttttg atttaatttc agcggccagc gcctggacct cgcgggcagc gtcgccctcg 4440
   ggttctgatt caagaacggt tgtgccggcg gcggcagtgc ctgggtagct cacgcgctgc 4500
   gtgatacggg actcaagaat gggcagctcg tacccggcca gcgcctcggc aacctcaccg 4560
   ccgatgcgcg tgcctttgat cgcccgcgac acgacaaagg ccgcttgtag ccttccatcc 4620
   gtgacctcaa tgcgctgctt aaccagctcc accaggtcgg cggtggccca tatgtcgtaa 4680
   gggcttggct gcaccggaat cagcacgaag tcggctgcct tgatcgcgga cacagccaag 4740
   tccgccgcct ggggcgctcc gtcgatcact acgaagtcgc gccggccgat ggccttcacg 4800
   tcgcggtcaa tcgtcgggcg gtcgatgccg acaacggtta gcggttgatc ttcccgcacg 4860
   gccgcccaat cgcgggcact gccctgggga tcggaatcga ctaacagaac atcggccccg 4920
   gcgagttgca gggcgcgggc tagatgggtt gcgatggtcg tcttgcctga cccgcctttc 4980
   tggttaagta cagcgataac cttcatgcgt tccccttgcg tatttgttta tttactcatc 5040
   gcatcatata cgcagcgacc gcatgacgca agctgtttta ctcaaataca catcaccttt 5100
   ttagacggcg gcgctcggtt tcttcagcgg ccaagctggc cggccaggcc gccagcttgg 5160
   catcagacaa accggccagg atttcatgca gccgcacggt tgagacgtgc gcgggcggct 5220
   cgaacacgta cccggccgcg atcatctccg cctcgatctc ttcggtaatg aaaaacggtt 5280
   cgtcctggcc gtcctggtgc ggtttcatgc ttgttcctct tggcgttcat tctcggcggc 5340
   cgccagggcg tcggcctcgg tcaatgcgtc ctcacggaag gcaccgcgcc gcctggcctc 5400
   ggtgggcgtc acttcctcgc tgcgctcaag tgcgcggtac agggtcgagc gatgcacgcc 5460
   aagcagtgca gccgcctctt tcacggtgcg gccttcctgg tcgatcagct cgcgggcgtg 5520
   cgcgatctgt gccggggtga gggtagggcg ggggccaaac ttcacgcctc gggccttggc 5580
   ggcctcgcgc ccgctccggg tgcggtcgat gattagggaa cgctcgaact cggcaatgcc 5640
   ggcgaacacg gtcaacacca tgcggccggc cggcgtggtg gtaacgcgtg gtgattttgt 5700
   gccgagctgc cggtcgggga gctgttggct ggctggtggc aggatatatt gtggtgtaaa 5760
   caaattgacg cttagacaac ttaataacac attgcggacg tctttaatgt actgaattaa 5820
   catccgtttg atacttgtct aaaattggct gatttcgagt gcatctatgc ataaaaacaa 5880
   tctaatgaca attattacca agcag 5905
<210> 8
   <211> 5027
   <212> DNA
   <213> Artificial
<400> 8
   ctgatcatga gcggagaatt aagggagtca cgttatgacc cccgccgatg acgcgggaca      60
   agccgtttta cgtttggaac tgacagaacc gcaacgttga aggagccact cagccgcggg     120
   tttctggagt ttaatgagct aagcacatac gtcagaaacc attattgcgc gttcaaaagt     180
   cgcctaaggt cactatcagc tagcaaatat ttcttgtcaa aaatgctcca ctgacgttcc     240
   ataaattccc ctcggtatcc aattagagtc tcatattcac tctcaatcca aataatctcg     300
   acatgtctcc ggagaggaga ccagttgaga ttaggccagc tacagcagcc gatatggccg     360
   cggtttgtga catcgttaac cattacattg agacgtctac agtgaacttt aggacagagc     420
   cacaaacacc acaagagtgg attgatgacc tagagaggtt gcaagataga tacccttggt     480
   tggttgctga ggttgagggt gttgtggctg gtattgctta cgctgggccc tggaaggcta     540
   ggaacgctta cgattggaca gttgagagta ctgtttacgt gtcacatagg catcaaaggt     600
   tgggcctagg atctacattg tacacacatt tgcttaagtc tatggaggcg caaggtttta     660
   agtctgtggt tgctgttata ggccttccaa acgatccatc tgttaggttg catgaggctt     720
   tgggatacac agcgcggggt acattgcgcg cggctggata caagcatggt ggatggcatg     780
   atgttggttt ttggcaaagg gattttgagt tgccagctcc tccaaggcca gttaggccag     840
   ttacccagat ctgagtcgat cgaccgatct tgctgcgttc ggatattttc gtggagttcc     900
   cgccacagac ccggatgatc cccgatcgtt caaacatttg gcaataaagt ttcttaagat     960
   tgaatcctgt tgccggtctt gcgatgatta tcatataatt tctgttgaat tacgttaagc    1020
   atgtaataat taacatgtaa tgcatgacgt tatttatgag atgggttttt atgattagag    1080
   tcccgcaatt atacatttaa tacgcgatag aaaacaaaat atagcgcgca aactaggata    1140
   aattatcgcg cgcggtgtca tctatgttac tagatcgggc ctgagtcgtt gtaaaacgac    1200
   ggccagtgaa ttatccaact ttgtataata aagttgccat gattacgcca agcttgcatg    1260
   ccgtcgacca gatctgatat ctgcggccgc ctcgagcata tgctagagga tccccgggta    1320
   ccccactttg tacaagaaag ctgggtggta ccgggccccc cctcgagttt aaaccactcc    1380
   gccctacact cgtatatata tgcctaaacc tgccccgttc ctcatatgtg atattattat    1440
   ttcattatta ggtataagat agtaaacgat aaggaaagac aatttattga gaaagccatg    1500
   ctaaaatata gatagatata ccttagcagg tgtttatttt acaacataac ataacatagt    1560
   agctagccag caggcaggct aaaacatagt atagtctatc tgcaggggaa ttcttagttc    1620
   accaactgac tcttcttcgt gccgtctgac tgtactcctg gaactgcagc atccagtgtc 1680
   tttgtgatgc gcttacacac gggtaactcc caccaagggg cataaggcca cctgtgatgc 1740
   tcccagtgat aatcaaagtg ataacatttc aagaatgaca gcggccgagg gtcatcagca 1800
   ctgtgagatt tctcccaggg cattgtctcc tgagcattgg aggggaggtg gggtaaatag 1860
   gtgccaaaat agaacaatcg gaacgcactg accaggggtg cagccgccag aaagacgcat 1920
   aaattggggt attgcacccg taaggattgt aggaagaagg ggtatgcgaa gatcttggca 1980
   aactggagtg gtgttgcata ctcccacata aacctgccaa accacttatg tagcgcagga 2040
   tcgcctcgat ggaagtctgg gtctttatgt ggtaacccag tgaagttgtg atgctcccag 2100
   tgcttcttgt gcagcatgtc atagtcaaac caggcatata ggctgatggc tatattgcca 2160
   agtaggtcat tcaaacgacg gttcctgatg gcaatagtcc catgcatggc atcatgcgtg 2220
   gtgatgaaaa gcccagtgtt gaggaactcc agtgccagaa acgttgcaac aacgtcccac 2280
   caggctgact tatcagggcc tgtgagtttg atttgccagc agccatggta aaagacagct 2340
   gtccataggg tgatgacagt gcatgcaatt gatacaccct tccagtcact gcgtggtggc 2400
   ctgtatttga gtgcggcttg aggtttggat actgtaccct tctcctccgg cataggatat 2460
   tgggctttcc acaagcttaa cgcgtcatcc tcatcatcgg ggccagccgg ggactgtaca 2520
   cgtggctgca catgtgtcac atctggcgcc atgcacttta ctcttccacc attgcttgta 2580
   atggaagtaa tgtcagtgtt gaccttctta actgggaatc cagtcatgga tttgaggccg 2640
   ccgaatggag ccaccgcggc cgattgcacc gtagaagcac ggctgactgt agtcacagct 2700
   gaagaggata tcatagaagc catttttctc acttctgtat gaattgcaaa gcttggatcc 2760
   tgaaatcgaa attcagagtt ttgatagtga gagcaaagag ggacggactt atgaggattt 2820
   cgagtatttc aagagatggt acttgttgat cggacggcta cgatgatctc gatttggtta 2880
   atccagtatc tcgcggtgta tggagttatg gtagggttaa tggtcaattt catctaacgg 2940
   tagagaatga tgtaattaga taagaatctt gagatactgg tttagattgg atgagtgtag 3000
   ggtccatctt atcttgataa gtggatggtt tttagagaca cagtgaatat tagccaatcg 3060
   aagttccata tcaccatcat catctgtata attttgtttt tttggaagat aataatgatt 3120
   gaaattttgg tagattttat ttttcattat ttaccttgta tgttgagtgg tcttcaaatt 3180
   attgaacgtg acagattcac aagaaagtag attttttata aatgaaattt tacttatttt 3240
   aaaggtatct ctatttaatt tcttttgttt atggttgtct gtcagcattt gacttgcagt 3300
   ttcatgctca tagtcatata cgttattcta ggcttttttg aatatcttat tacttttttc 3360
   gtaatacaat tttataattt tatcaaagtt atacaactat aactaaaatt agggttttct 3420
   acaaaacaaa aaaatcttct aatttttttt gttgtagcca gtttactcgt aagttacaaa 3480
   aaaatacaaa tgaacccaca tgtattatgc gtttaactag gattaccatg tactttcatg 3540
   tactcaattc accctatact cttttttttt ttttttctag ttccacccaa tctataaaat 3600
   tctgtccatt tgaccaaatt caattaattt ctgtaattgc gatttaaaat taatattaca 3660
   tgttcactat ttctcgattt gagggaaccc gagtttaaat atgataaaaa tgttgaccca 3720
   tcactacaaa tatgttatag tttatactta atagtggtgt ttttggggat aattgatgaa 3780
   ttaagtaaac atgattcttc ttatgaagtt gattgagtga ttattgtatg taaacctatg 3840
   tgattgatgt tattggttga ttgagtgatt attgtattag tatgtaagca aagatgattg 3900
   ttcttatgag gtaatttgtt actcattcat ccttttgcat atgagaaatt gtgttagcgt 3960
   acgcaaaaca atagagaaca taaaagatat gtgtatttat ttaaggtgac ttttgttaat 4020
   gatattgtag tatctataca tttatatata acttgttgaa tttgagtata agctatcatt 4080
   ttctaattat tggttgaatt tatactttaa aattaggtgc aaatatatta gatgagtagt 4140
   ccattgatct aaatatttcg cataataatt ttaagtacta aaatattgtt gacatatttt 4200
   ataattttta aactttaaat tttttatata agcttttgtt gacacttaat tcgtttaaat 4260
   ttggttaata ctgtaataat atgtactttg ttgcctatta gagagacacc ctctaaatcg 4320
   tccagagact cttctctttg cagctaaagt ctcccatttc ataaacccta gccaaaactg 4380
   gtgagcgatt cttctccggc aatttgatta accttctagt tcgtaacgag tttcattgtt 4440
   gcttccttca cttttggaac tcgtatgttt tgaagatcaa atgatttttc tggtctgtgt 4500
   ttctttcatt tggtttgggc ctatcgaaat cgtttatgca cacaacctgt tcgacgtagt 4560
   gcgtagctga gattcttctc tataaaaagg tgtattcttt gctattgttg gaaggtatca 4620
   ttgtttagta atgggtaaca aagtgatgat gttcaagtgg tcaaagaaca tcacgccttc 4680
   acaggttatc aaactgatgc gagctgaaaa agatgtggag aagtctatgg cagtttttga 4740
   ttccgcaacg gcggaatacg caaatgggta cgtacatgat cagagttctt ttggttatga 4800
   gatcagatct ggtcgacggc atgcaagctt ggcgtaatca tggagcctgc ttttttgtac 4860
   aaacttgcca tgattacgcc aagcttgcat gccgtcgacc agatctgata tctgcggccg 4920
   cctcgagcat atgctagagg atccccgggt acccaacttt tctatacaaa gttggatggc 4980
   atgcaagctt ggcgtaatca tggtcatagc tgtttcctac tagatct 5027
<210> 9
   <211> 4157
   <212> DNA
   <213> Artificial
<400> 9
   ctgatcatga gcggagaatt aagggagtca cgttatgacc cccgccgatg acgcgggaca 60
   agccgtttta cgtttggaac tgacagaacc gcaacgttga aggagccact cagccgcggg 120
   tttctggagt ttaatgagct aagcacatac gtcagaaacc attattgcgc gttcaaaagt 180
   cgcctaaggt cactatcagc tagcaaatat ttcttgtcaa aaatgctcca ctgacgttcc 240
   ataaattccc ctcggtatcc aattagagtc tcatattcac tctcaatcca aataatctcg 300
   acatgtctcc ggagaggaga ccagttgaga ttaggccagc tacagcagcc gatatggccg 360
   cggtttgtga catcgttaac cattacattg agacgtctac agtgaacttt aggacagagc 420
   cacaaacacc acaagagtgg attgatgacc tagagaggtt gcaagataga tacccttggt 480
   tggttgctga ggttgagggt gttgtggctg gtattgctta cgctgggccc tggaaggcta 540
   ggaacgctta cgattggaca gttgagagta ctgtttacgt gtcacatagg catcaaaggt 600
   tgggcctagg atctacattg tacacacatt tgcttaagtc tatggaggcg caaggtttta 660
   agtctgtggt tgctgttata ggccttccaa acgatccatc tgttaggttg catgaggctt 720
   tgggatacac agcgcggggt acattgcgcg cggctggata caagcatggt ggatggcatg 780
   atgttggttt ttggcaaagg gattttgagt tgccagctcc tccaaggcca gttaggccag 840
   ttacccagat ctgagtcgat cgaccgatct tgctgcgttc ggatattttc gtggagttcc 900
   cgccacagac ccggatgatc cccgatcgtt caaacatttg gcaataaagt ttcttaagat 960
   tgaatcctgt tgccggtctt gcgatgatta tcatataatt tctgttgaat tacgttaagc 1020
   atgtaataat taacatgtaa tgcatgacgt tatttatgag atgggttttt atgattagag 1080
   tcccgcaatt atacatttaa tacgcgatag aaaacaaaat atagcgcgca aactaggata 1140
   aattatcgcg cgcggtgtca tctatgttac tagatcgggc ctgagtcgtt gtaaaacgac 1200
   ggccagtgaa ttatccaact ttgtataata aagttgccat gattacgcca agcttgcatg 1260
   ccgtcgacca gatctgatat ctgcggccgc ctcgagcata tgctagagga tccccgggta 1320
   ccccactttg tacaagaaag ctgggtggta ccgggccccc cctcgagttt aaaccactcc 1380
   gccctacact cgtatatata tgcctaaacc tgccccgttc ctcatatgtg atattattat 1440
   ttcattatta ggtataagat agtaaacgat aaggaaagac aatttattga gaaagccatg 1500
   ctaaaatata gatagatata ccttagcagg tgtttatttt acaacataac ataacatagt 1560
   agctagccag caggcaggct aaaacatagt atagtctatc tgcatgctcg agcggccgcc 1620
   agtgtgatgg atatctgcag aattcgccct tcggtcgact tactccacta ctgctcctgc 1680
   aggggccagc tgggagacaa cctgcgcttt tgcctgctgc atgggctgct gctgcttggt 1740
   ttgttggtga tgctgagttt gttgtgtaat tttcttcgcc ttcggcaact cccaccaggg 1800
   agcatatggc catctgtggt gttcccaatg ataatcaaag tggtaacact tgaggaaaga 1860
   cagccaggag ggagcatcac tagagtgact cttctgccac accatcacct cctgggcgtt   1920
   ggggggcaag tgcggcaggt aggtgccaaa atagaacagc ctgaaggcgg ccaccagggg   1980
   cgcagcagcc atgtagacac acaggttgtt gtagggcact cccagggctt gcagcacctg   2040
   actcaccagg atgattttgg caaactgcaa gggggtggag tattcccaca tgaacctggc   2100
   ataccacact ggcagtgcag ggttgcccct gtggaagtca gggtctttgc ccttctgtcc   2160
   tgtgtagttg tgatgctccc agtgcttcct gtgaagcatg tcgtagtcaa accaggcgta   2220
   cagtgtgatg gctatacgac ccaacagatc gttcagcttg cggttcctgt aacaaacagt   2280
   gccatgcatg gcatcgtgcg tggtgatgaa aaggcctgtg ttgacgaact ccaacaaaat   2340
   aaatgtgcca gcaatgtcaa tccaccagga gcgctgactg ccagacagct ggatctgcca   2400
   gcagccgtgg taaaacagca cgcaccaggc tgcaatcagg cccaaggcaa tgcttatgcc   2460
   cttcacatca ttgcgaggtg gcctgtacaa ctgcttgacc tcattcatgc tccctgctac   2520
   attttcatcc ggcagagggt attgcgcttt ccacacgtcg atgacatcgg gctcagggat   2580
   ggcagcatct gagccactgg ttggagagcc agctttggtc tgctgcggca gcgttgattg   2640
   ccgcctggga gccatgcact ttactcttcc accattgctt gtaatggaag taatgtcagt   2700
   gttgaccttc ttaactggga atccagtcat ggatttgagg ccgccgaatg gagccaccgc   2760
   ggccgattgc accgtagaag cacggctgac tgtagtcaca gctgaagagg atatcataga   2820
   agccattttt ctcacttctg tatgaattgc aaagctatct cgaagctagt gaacaagaaa   2880
   aatgtacata cactgactac taatttatag ccatcattac tgctgatttg gcaaatggcg   2940
   taatctcaaa ccaaatcacg aggggtaata aatgttagcc acataaattg gtaagatcaa   3000
   aggacgatat tcagacatct taagcatcgt ggtgtgaggc gataattgtt ttgggctttt   3060
   tggctcttgt ttatggcaag aaaatccaat ttgacacatg ctggctaatt ttcaaaaagc   3120
   ataaaggatt agtcagggat tctgaaacta ggagtgcaat tttacacaca gaaaaatcac   3180
   ataatcgtaa cctataatca cgatgtgatt caatgccatg attatatctt caaactgaaa   3240
   tcatagagct cattaaattg ttgtcttttg ttatatatta atggtacata atgcccttta   3300
   aatgcttttg tactttgcga aattttgaaa taaacaaaat acattgtttc tgtatgcaca   3360
   tggaaagtac taataaacga aaaagaaagt ttctctgcaa aaaacaaaaa aaaatatatt   3420
   tagggataga cagaaatcat tgtttaggaa agttgctctt tattatctct actacttctc   3480
   cacgagaatt aggcgccatg caaatgacac cgatgctaga aacttcggca gtgtactatt   3540
   gtctggccgt tttctgatta tcgctcagtt tgccaactac tttggtcctt taacggtcga   3600
   gatctgctac catgcgataa atatcttgac agcgttttgg aaagcgcgat tgagaggaaa   3660
   cgtccaaact atttgtttat tgggcttata attgactaaa aatgggcctt gttaacagaa   3720
   tctcttgtat tttttttctt ttgaaattta catcactttt ccataacaaa cgaaatgcaa   3780
   caaactgtaa aaaaactttg tgttagatgg agaatttaca gccattcgat tggtttcaaa   3840
   atatgtcaag agtctcaatg atcggacggt tgcaaagagg acacgagcct ctgcagcgca   3900
   agggcgaatt ccagcacact ggcggccgtt actagtggat ccgagctcgg taccaagctt   3960
   ggcgtaatca tggagcctgc ttttttgtac aaacttgcca tgattacgcc aagcttgcat   4020
   gccgtcgacc agatctgata tctgcggccg cctcgagcat atgctagagg atccccgggt   4080
   acccaacttt tctatacaaa gttggatggc atgcaagctt ggcgtaatca tggtcatagc   4140
   tgtttcctac tagatct                                                  4157
<210> 10
   <211> 960
   <212> DNA
   <213> Brassica napus
<400> 10
   ggtaccaggc tcgtgtcctc tttgcaaccg tccgatcatt gagatcttag acatattttg     60
   aaaccaatcg aatggctgta aattctcgat ctaacacagt ttatacattc gtttgttatg    120
   ggaagatgat gtaaatttca aaaaaataca agatattctg ttcagaaggc ccattaagtc    180
   aattttaagc ccaataaacc aatagtttgg acgtttcctc tctatcgcgc ttttccgaaa    240
   cgccagaaaa acgctgtcaa gatatttatc gcatggtagc agatctcgac cgttaaagga    300
   ccaaagtagt tggcaaactg agcgataatc agaaaacagc cagacaatag tacaccgccg    360
   aagtttctag catcgtgtca tttgcatggc gcctaattct cgtcgagaac tagagataat    420
   aaagagcaac tttcctaaac aataatttct gtctatccct aaatattttt ttttgttctt    480
   tgcagagaaa ctttcttttt catttattag tactttccat gtgcacagag aaacaatgta    540
   ttttgtttac ttcaaaattt cacaaagtac aaaagcattt aaaggacatt atgtgacaca    600
   cgtcatacaa tttaatgagc tctatgactt tcagtttgaa gatctaatta tagcattgaa    660
   tcacatcgta attacagatt atgattatct gtgtgtaaaa ttgtactcct agtttcagaa    720
   tcccttacta atcaatcctt tctacatttt gaaaaattag tcagcatgtg tcaaattgga    780
   tttcttgcca taaacaagag ccaaaaaaaa cccaaaacaa tgatcgcctc acaccacgat    840
   gcttaagatg tctgtctgat tatcgtgcct ttgatcttac caatttatgt ggctaacatt    900
   tattacccct cgtgatttgg tttgagatta cgccatttgc caaataagca gtaaaagctt    960
<210> 11
   <211> 1043
   <212> DNA
   <213> Brassica napus
<400> 11
   ggtaccaggc tcgtgtcctc tttgcaaccg tccgatcatt gagactcttg acatattttg     60
   aaaccaatcg aatggctgta aattctccat ctaacacaaa gtttttttac agtttgttgc    120
   atttcgtttg ttatggaaaa gtgatgtaaa tttcaaaaga aaaaaaatac aagagattct    180
   gttaacaagg cccattttta gtcaattata agcccaataa acaaatagtt tggacgtttc    240
   ctctcaatcg cgctttccaa aacgctgtca agatatttat cgcatggtag cagatctcga    300
   ccgttaaagg accaaagtag ttggcaaact gagcgataat cagaaaacgg ccagacaata    360
   gtacactgcc gaagtttcta gcatcggtgt catttgcatg gcgcctaatt ctcgtggaga    420
   agtagtagag ataataaaga gcaactttcc taaacaatga tttctgtcta tccctaaata    480
   tatttttttt tgttttttgc agagaaactt tctttttcgt ttattagtac tttccatgtg    540
   catacagaaa caatgtattt tgtttatttc aaaatttcgc aaagtacaaa agcatttaaa    600
   gggcattatg taccattaat atataacaaa agacaacaat ttaatgagct ctatgatttc    660
   agtttgaaga tataatcatg gcattgaatc acatcgtgat tataggttac gattatgtga    720
   tttttctgtg tgtaaaattg cactcctagt ttcagaatcc ctgactaatc ctttatgctt    780
   tttgaaaatt agccagcatg tgtcaaattg gattttcttg ccataaacaa gagccaaaaa    840
   gcccaaaaca attatcgcct cacaccacga tgcttaagat gtctgaatat cgtcctttga    900
   tcttaccaat ttatgtggct aacatttatt acccctcgtg atttggtttg agattacgcc    960
   atttgccaaa tcagcagtaa tgatggctat aaattagtag tcagtgtatg tacatttttc   1020
   ttgttcacta gcttcgaaag ctt                                           1043